(19) Europäisches Patentamt
European Patent Office
Office européen des brevets

(11) EP 4 768 032 A1

(12) **EUROPEAN PATENT APPLICATION**
published in accordance with Art. 153(4) EPC

(43) Date of publication:
**01.07.2026 Bulletin 2026/27**

(21) Application number: **24855885.0**

(22) Date of filing: **23.08.2024**

(51) International Patent Classification (IPC):
***A61K 31/713*** *(2006.01)* ***C12N 15/113*** *(2010.01)*
***A61P 11/00*** *(2006.01)*

(52) Cooperative Patent Classification (CPC):
**A61K 31/713; A61P 11/00; C12N 15/113**

(86) International application number:
**PCT/CN2024/114147**

(87) International publication number:
**WO 2025/040164 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.08.2023 CN 202311070972**
**18.01.2024 CN 202410074707**

(71) Applicant: **Tuojie Biotech (Shanghai) Co., Ltd.**
**Shanghai 201203 (CN)**

(72) Inventors:
- **LIU, Min**
  **Shanghai 201203 (CN)**
- **LIN, Xiaoyan**
  **Shanghai 201203 (CN)**
- **LI, Yunfei**
  **Shanghai 201203 (CN)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

# (54) RAGE-TARGETED RNAI AGENT AND PHARMACEUTICAL USE THEREOF

(57) The present invention relates to a RAGE-targeted RNAi agent and a pharmaceuti cal use thereof. Specifically, the present invention relates to a RAGE-targeted RN Ai agent, a pharmaceutical composition, and a pharmaceutical use thereof. The p resent invention further relates to a pharmaceutical composition, cell or kit compr ising the RNAi agent, and a method for using the RNAi agent to treat and/or pr event related disorders of a subject.

EP 4 768 032 A1

## Description

The present disclosure claims priority to Chinese Patent Application No. 202311070972.3 filed on August 24, 2023 and Chinese Patent Application No. 202410074707.0 filed on January 18, 2024, which are incorporated herein by reference in their entirety.

## TECHNICAL FIELD

The present disclosure pertains to the field of biopharmaceuticals and particularly relates to an RNAi agent targeting the receptor for advanced glycosylation end-products (RAGE or AGER or "advanced glycosylation end-product specific receptor"), a pharmaceutical composition, and pharmaceutical use thereof.

## BACKGROUND

The receptor for advanced glycosylation end-products (RAGE or AGER) is a pro-inflammatory pattern receptor. It possesses a short intracellular domain, a transmembrane domain, and an extracellular domain consisting of three immunoglobulin-like domains. RAGE is widely distributed in many tissues and cell types. It is expressed during embryonic development, and its expression level is down-regulated in adulthood and then up-regulated in old age. Normally, RAGE is expressed at low levels in most healthy cells. However, it is highly expressed in the lungs throughout life. RAGE is associated with chronic pathological inflammation, which can lead to many diseases, including pulmonary diseases, cardiovascular diseases, cancer, diabetes, chronic kidney disease, neurodegenerative diseases, rheumatoid arthritis, nonalcoholic steatohepatitis, injuries caused by certain viral infections, etc.

In the field of pulmonary diseases, RAGE gene knockout mice are physiologically and histologically completely free from allergic asthma caused by dust mite allergens or ovalbumin. Similarly, RAGE gene knockout mice are free from acute lung injury and inflammation induced by hyperoxia or lipopolysaccharides. In addition, the expression level of RAGE is significantly up-regulated in tissues affected by allergic airway inflammation. Genome-wide association studies (GWAS) have linked a gain-of-function mutation allele of RAGE (G82S) to increased inflammation, reduced lung function, and asthma risk, suggesting that knocking down RAGE has a therapeutic effect on diseases such as pulmonary inflammation or asthma.

## SUMMARY

The present disclosure provides an RNAi agent (RNA interference agent) targeting RAGE. In some embodiments, the present disclosure provides an RNAi agent comprising a sense strand and an antisense strand that form a double-stranded region, wherein:

the sense strand comprises a sequence differing from any one of the nucleotide sequences of SEQ ID NO: 1 to SEQ ID NO: 8, SEQ ID NO: 53 to SEQ ID NO: 106, SEQ ID NO: 275, and SEQ ID NO: 277 by no more than 3 (e.g., 0, 1, 2, or 3) nucleotides, and comprises at least 15 (e.g., 16, 17, 18, 19, 20, or 21) contiguous nucleotides;
the antisense strand comprises a sequence differing from any one of the nucleotide sequences of SEQ ID NO: 27 to SEQ ID NO: 34, SEQ ID NO: 107 to SEQ ID NO: 160, SEQ ID NO: 276, and SEQ ID NO: 278 by no more than 3 (e.g., 0, 1, 2, or 3) nucleotides, and comprises at least 15 (e.g., 16, 17, 18, 19, 20, or 21) contiguous nucleotides.

In some embodiments, the antisense strand is at least partially reverse complementary to a target sequence to mediate RNA interference. In some embodiments, there are no more than 5, no more than 4, no more than 3, no more than 2, or no more than 1 mismatch between the antisense strand and the target sequence. In some embodiments, the antisense strand is fully reverse complementary to the target sequence.

In some embodiments, the sense strand is at least partially reverse complementary to the antisense strand to form a double-stranded region. In some embodiments, there are no more than 5, no more than 4, no more than 3, no more than 2, or no more than 1 mismatch between the sense strand and the antisense strand. In some embodiments, the sense strand is fully reverse complementary to the antisense strand.

In some embodiments, the RNAi agent of the present disclosure comprises one or two blunt ends.

In some specific embodiments, each strand of the RNAi agent independently comprises an overhang comprising 1 to 2 unpaired nucleotides.

In some embodiments, the RNAi agent of the present disclosure comprises an overhang at the 3' end of the antisense strand of the RNAi agent.

In some embodiments, the sense strand and the antisense strand each independently have 16 to 35, 16 to 34, 17 to 34, 17 to 33, 18 to 33, 18 to 32, 18 to 31, 18 to 30, 18 to 29, 18 to 28, 18 to 27, 18 to 26, 18 to 25, 18 to 24, 18 to 23, 19 to 25,

19 to 24, or 19 to 23 nucleotides (e.g., 19, 20, 21, 22, or 23 nucleotides).

In some embodiments, the sense strand and the antisense strand are identical or different in length; the sense strand is 19-23 nucleotides in length, and the antisense strand is 19-26 nucleotides in length. The length ratio of the sense strand to the antisense strand of the RNAi agent provided by the present disclosure may be 19/19, 19/20, 19/21, 19/22, 19/23, 19/24, 19/25, 19/26, 20/19, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25, or 23/26. In some embodiments, the length ratio of the sense strand to the antisense strand of the RNAi agent is 19/21, 21/23, or 23/25. In some specific embodiments, the length ratio of the sense strand to the antisense strand of the RNAi agent is 19/21.

In some embodiments, the sense strand comprises at least 15 contiguous nucleotides and differs from any one of the nucleotide sequences of SEQ ID NO: 1 to SEQ ID NO: 8, SEQ ID NO: 53 to SEQ ID NO: 106, SEQ ID NO: 275, and SEQ ID NO: 277 by no more than 2 nucleotides; in some embodiments, by no more than one nucleotide; and in some embodiments, by one nucleotide.

In some embodiments, the antisense strand comprises a sequence of at least 15 contiguous nucleotides and differs from the nucleotide sequences of SEQ ID NO: 27 to SEQ ID NO: 34, SEQ ID NO: 107 to SEQ ID NO: 160, SEQ ID NO: 276, and SEQ ID NO: 278 by no more than 2 nucleotides; in some embodiments, by no more than one nucleotide; and in some embodiments, by one nucleotide.

In some embodiments, the sense strand comprises at least 15 contiguous nucleotides of any one of the nucleotide sequences of SEQ ID NO: 1 to SEQ ID NO: 8, SEQ ID NO: 53 to SEQ ID NO: 106, SEQ ID NO: 275, and SEQ ID NO: 277. In some embodiments, the sense strand comprises at least 16 contiguous nucleotides of any one of the nucleotide sequences of SEQ ID NO: 1 to SEQ ID NO: 8, SEQ ID NO: 53 to SEQ ID NO: 106, SEQ ID NO: 275, and SEQ ID NO: 277. In some embodiments, the sense strand comprises at least 17 contiguous nucleotides of any one of the nucleotide sequences of SEQ ID NO: 1 to SEQ ID NO: 8, SEQ ID NO: 53 to SEQ ID NO: 106, SEQ ID NO: 275, and SEQ ID NO: 277. In some embodiments, the sense strand comprises at least 19 contiguous nucleotides of any one of the nucleotide sequences of SEQ ID NO: 1 to SEQ ID NO: 8, SEQ ID NO: 53 to SEQ ID NO: 106, SEQ ID NO: 275, and SEQ ID NO: 277. In some embodiments, the sense strand comprises at least 18 contiguous nucleotides of any one of the nucleotide sequences of SEQ ID NO: 1 to SEQ ID NO: 8, SEQ ID NO: 53 to SEQ ID NO: 106, SEQ ID NO: 275, and SEQ ID NO: 277.

In some embodiments, the antisense strand comprises at least 15 contiguous nucleotides of any one of the nucleotide sequences of SEQ ID NO: 27 to SEQ ID NO: 34, SEQ ID NO: 107 to SEQ ID NO: 160, SEQ ID NO: 276, and SEQ ID NO: 278. In some embodiments, the antisense strand comprises at least 17 contiguous nucleotides of any one of the nucleotide sequences of SEQ ID NO: 27 to SEQ ID NO: 34, SEQ ID NO: 107 to SEQ ID NO: 160, SEQ ID NO: 276, and SEQ ID NO: 278. In some embodiments, the antisense strand comprises at least 19 contiguous nucleotides of any one of the nucleotide sequences of SEQ ID NO: 27 to SEQ ID NO: 34, SEQ ID NO: 107 to SEQ ID NO: 160, SEQ ID NO: 276, and SEQ ID NO: 278. In some embodiments, the antisense strand comprises at least 20 contiguous nucleotides of any one of the nucleotide sequences of SEQ ID NO: 27 to SEQ ID NO: 34, SEQ ID NO: 107 to SEQ ID NO: 160, SEQ ID NO: 276, and SEQ ID NO: 278. In some embodiments, the antisense strand comprises at least 21 contiguous nucleotides of any one of the nucleotide sequences of SEQ ID NO: 27 to SEQ ID NO: 34, SEQ ID NO: 107 to SEQ ID NO: 160, SEQ ID NO: 276, and SEQ ID NO: 278.

In some embodiments, the sense strand comprises or is selected from the group consisting of any one of the following nucleotide sequences: SEQ ID NO: 1 to SEQ ID NO: 8, SEQ ID NO: 53 to SEQ ID NO: 106, SEQ ID NO: 275, and SEQ ID NO: 277.

In some embodiments, the antisense strand comprises or is selected from the group consisting of any one of the following nucleotide sequences: SEQ ID NO: 27 to SEQ ID NO: 34, SEQ ID NO: 107 to SEQ ID NO: 160, SEQ ID NO: 276, and SEQ ID NO: 278. In some embodiments, the RNAi agent comprises or is selected from the group consisting of any one of the following groups:

group 1): a sense strand set forth in SEQ ID NO: 1 and an antisense strand set forth in SEQ ID NO: 27;
group 2): a sense strand set forth in SEQ ID NO: 7 and an antisense strand set forth in SEQ ID NO: 33;
group 3): a sense strand set forth in SEQ ID NO: 2 and an antisense strand set forth in SEQ ID NO: 28;
group 4): a sense strand set forth in SEQ ID NO: 8 and an antisense strand set forth in SEQ ID NO: 34;
group 5): a sense strand set forth in SEQ ID NO: 3 and an antisense strand set forth in SEQ ID NO: 29;
group 6): a sense strand set forth in SEQ ID NO: 4 and an antisense strand set forth in SEQ ID NO: 30;
group 7): a sense strand set forth in SEQ ID NO: 5 and an antisense strand set forth in SEQ ID NO: 31; and
group 8): a sense strand set forth in SEQ ID NO: 6 and an antisense strand set forth in SEQ ID NO: 32.

In some embodiments, at least one nucleotide in the sense strand and/or the antisense strand is a modified nucleotide.

In some embodiments, all nucleotides in the sense strand and/or the antisense strand are modified nucleotides.

In some embodiments, three contiguous nucleotides in the sense strand of the RNAi agent are 2'-fluoro-modified nucleotides.

In some embodiments, in the direction from the 5' end to the 3' end, the three contiguous nucleotides at positions 7-9 of the sense strand are 2'-fluoro-modified nucleotides.

In some embodiments, in the direction from the 5' end to the 3' end, the three contiguous nucleotides at positions 7-9 of the sense strand are 2'-fluoro-modified nucleotides, and the nucleotides at the remaining positions of the sense strand are all 2'-methoxy-modified nucleotides.

In some embodiments, in the direction from the 5' end to the 3' end, each of the nucleotides at positions 2, 6, 12, 14, and 16 of the antisense strand is independently a 2'-fluoro-modified nucleotide. In some embodiments, the nucleotide at position 2, 4, 6, 10, 12, 14, 16, or 18 of the antisense strand is independently a 2'-fluoro-modified nucleotide. In some embodiments, the nucleotides at the remaining positions of the antisense strand are all 2'-methoxy-modified nucleotides.

In some embodiments, the 5' end of the antisense strand comprises one phosphorus-containing group, and the phosphorus-containing group is a 5'-vinylphosphodiester group (5'-VP). When the 5'-end phosphorus-containing group is a 5'-vinylphosphodiester group (5'-VP), the 5'-VP may be a 5'-E-VP isomer (i.e., a trans-vinylphosphodiester group), a 5'-Z-VP isomer (i.e., a cis-vinylphosphodiester group), or a mixture thereof.

In some embodiments, the 5' end of the antisense strand comprises one phosphorus-containing group, and the phosphorus-containing group is a 5'-E-VP isomer (i.e., a trans-vinylphosphodiester group).

In some embodiments, the 5' end of the antisense strand comprises one phosphorus-containing group, and the phosphorus-containing group is

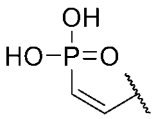 or 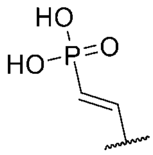

or a mixed form thereof.

In some embodiments, the 5' end of the antisense strand comprises one phosphorus-containing group, and the phosphorus-containing group is

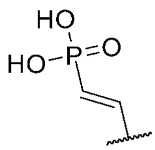.

In some embodiments, at least one phosphodiester group in the sense strand and/or the antisense strand is a phosphodiester group with a modification group. The modification group causes the RNAi agent to have increased stability in a biological sample or environment. In some embodiments, the phosphodiester group with a modification group is a phosphorothioate diester group. In some embodiments, the phosphodiester group with a modification group is a 5'-vinylphosphodiester group.

In some embodiments, the phosphorothioate diester group is present in at least one of the following positions:

between the 1st and 2nd nucleotides of the 5' end of the sense strand;
between the 2nd and 3rd nucleotides of the 5' end of the sense strand;
between the 1st and 2nd nucleotides of the 3' end of the sense strand;
between the 2nd and 3rd nucleotides of the 3' end of the sense strand;
between the 1st and 2nd nucleotides of the 5' end of the antisense strand;
between the 2nd and 3rd nucleotides of the 5' end of the antisense strand;
between the 1st and 2nd nucleotides of the 3' end of the antisense strand; and
between the 2nd and 3rd nucleotides of the 3' end of the antisense strand.

In some embodiments, the sense strand and/or the antisense strand comprise(s) a plurality of phosphorothioate diester groups, and the phosphorothioate diester groups are present:

between the 1st and 2nd nucleotides of the 5' end of the sense strand; and
between the 2nd and 3rd nucleotides of the 5' end of the sense strand; and
between the 1st and 2nd nucleotides of the 5' end of the antisense strand; and

between the 2nd and 3rd nucleotides of the 5' end of the antisense strand; and
between the 1st and 2nd nucleotides of the 3' end of the antisense strand; and
between the 2nd and 3rd nucleotides of the 3' end of the antisense strand.

In some embodiments, the sense strand and/or the antisense strand comprise(s) a plurality of phosphorothioate diester groups, and the phosphorothioate diester groups are present:

between the 1st and 2nd nucleotides of the 5' end of the sense strand; and
between the 2nd and 3rd nucleotides of the 5' end of the sense strand; and
between the 1st and 2nd nucleotides of the 3' end of the sense strand; and
between the 2nd and 3rd nucleotides of the 3' end of the sense strand; and
between the 1st and 2nd nucleotides of the 5' end of the antisense strand; and
between the 2nd and 3rd nucleotides of the 5' end of the antisense strand; and
between the 1st and 2nd nucleotides of the 3' end of the antisense strand; and
between the 2nd and 3rd nucleotides of the 3' end of the antisense strand.

In some embodiments, the 5'-vinylphosphodiester group is present at the 5' end of the antisense strand.
In some embodiments, the sense strand is selected from the group consisting of or comprises the nucleotide sequence set forth in any one of SEQ ID NO: 9 to SEQ ID NO: 20 and SEQ ID NO: 161 to SEQ ID NO: 214.
In some embodiments, the antisense strand is selected from the group consisting of or comprises the nucleotide sequence set forth in any one of SEQ ID NO: 35 to SEQ ID NO: 46 and SEQ ID NO: 215 to SEQ ID NO: 268.
In some embodiments, the RNAi agent comprises or is selected from the group consisting of any one of the following groups:

group 1): a sense strand set forth in SEQ ID NO: 9 and an antisense strand set forth in SEQ ID NO: 35;
group 2): a sense strand set forth in SEQ ID NO: 10 and an antisense strand set forth in SEQ ID NO: 36;
group 3): a sense strand set forth in SEQ ID NO: 11 and an antisense strand set forth in SEQ ID NO: 37;
group 4): a sense strand set forth in SEQ ID NO: 12 and an antisense strand set forth in SEQ ID NO: 38;
group 5): a sense strand set forth in SEQ ID NO: 13 and an antisense strand set forth in SEQ ID NO: 39;
group 6): a sense strand set forth in SEQ ID NO: 14 and an antisense strand set forth in SEQ ID NO: 40;
group 7): a sense strand set forth in SEQ ID NO: 15 and an antisense strand set forth in SEQ ID NO: 41;
group 8): a sense strand set forth in SEQ ID NO: 16 and an antisense strand set forth in SEQ ID NO: 42;
group 9): a sense strand set forth in SEQ ID NO: 17 and an antisense strand set forth in SEQ ID NO: 43;
group 10): a sense strand set forth in SEQ ID NO: 18 and an antisense strand set forth in SEQ ID NO: 44;
group 11): a sense strand set forth in SEQ ID NO: 19 and an antisense strand set forth in SEQ ID NO: 45; and
group 12): a sense strand set forth in SEQ ID NO: 20 and an antisense strand set forth in SEQ ID NO: 46.

In some embodiments, the RNAi agent further comprises a targeting ligand.
In some embodiments, the RNAi agent and the targeting ligand are linked covalently or non-covalently.
In some embodiments, the targeting ligand is linked to the antisense strand of the RNAi agent. In some embodiments, the targeting ligand is linked to the 5' end of the antisense strand of the RNAi agent. In some embodiments, the targeting ligand is linked to the 3' end of the antisense strand of the RNAi agent.
In some embodiments, the targeting ligand is linked to the sense strand of the RNAi agent.
In some embodiments, the targeting ligand is linked to the 5' end of the sense strand of the RNAi agent. In some embodiments, the targeting ligand is linked to the 3' end of the sense strand of the RNAi agent.
In some embodiments, the targeting ligand is linked to the 3' end or the 5' end of the sense strand by a phosphodiester group, a phosphorothioate diester group, or a phosphonic acid group.
In some embodiments, the targeting ligand is linked to the 3' end or the 5' end of the sense strand by a phosphodiester group. In some embodiments, the targeting ligand is linked to the 5' end of the sense strand by a phosphodiester group.
In some embodiments, the targeting ligand is indirectly linked to the 3' end or the 5' end of the sense strand by a phosphodiester group, a phosphorothioate diester group, or a phosphonic acid group.
In some embodiments, the targeting ligand is indirectly linked to the 3' end or the 5' end of the sense strand by a phosphodiester group.
In some embodiments, the targeting ligand is indirectly linked to the 5' end of the sense strand by a phosphodiester group.
In some embodiments, the targeting ligand is directly linked to the 3' end or the 5' end of the sense strand by a phosphodiester group, a phosphorothioate diester group, or a phosphonic acid group.

In some embodiments, the targeting ligand is directly linked to the 3' end or the 5' end of the sense strand by a phosphodiester group. In some embodiments, the targeting ligand is directly linked to the 5' end of the sense strand by a phosphodiester group.

In some embodiments, the targeting ligand may comprise 2, 3, 4, or more than 4 targeting groups. In some embodiments, the targeting ligand described in the present disclosure may comprise 1, 2, 3, 4, or more than 4 targeting groups linked to the 3' end or the 5' end of the sense strand by a linker.

In some embodiments, the targeting ligand targets the lungs.

In some embodiments, the targeting ligand has affinity for a cellular receptor expressed on an epithelial cell. In some embodiments, the targeting ligand includes an integrin-targeting ligand. In some embodiments, the integrin-targeting ligand is an αvβ6 integrin-targeting ligand.

In some embodiments, the targeting ligand has the following structure:

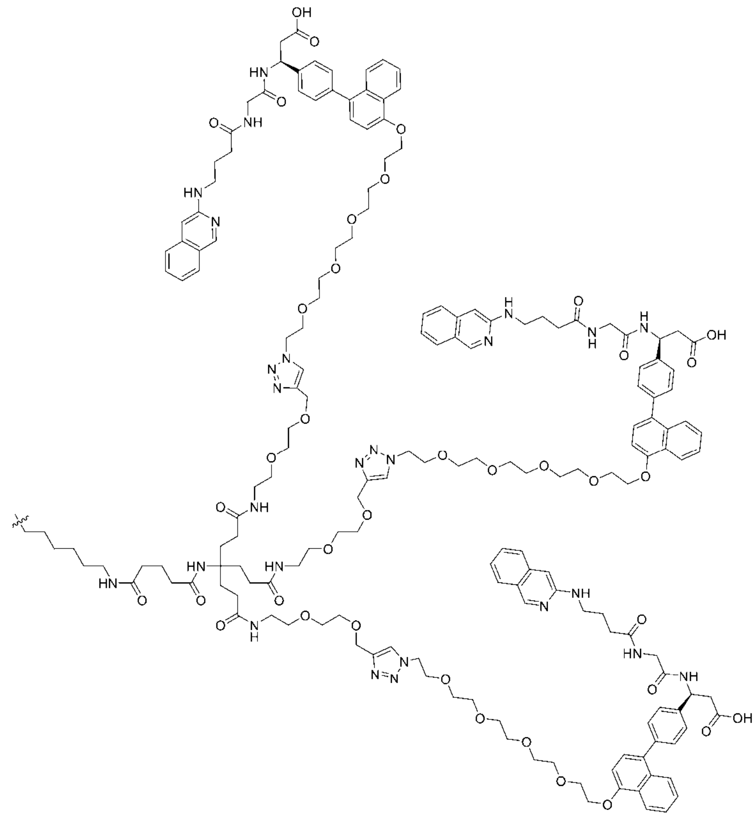

(formula **I-14**).

In some embodiments, the targeting ligand consists of one or more targeting groups, and the targeting ligand assists in directing the delivery of a therapeutic agent linked thereto to the desired target location. In some cases, the targeting ligand may bind to a cell or cellular receptor and initiate endocytosis to promote entry of the therapeutic agent into the cell. The targeting ligand may comprise a compound with affinity for a cellular receptor or a cell surface molecule or an antibody. Various targeting ligands comprising targeting groups may be linked to therapeutic agents and other compounds to deliver the agents to cells and specific cellular receptors in a targeted manner.

In some embodiments, each targeting group comprises a galactosamine derivative that is N-acetyl-galactosamine. Other sugars that may be used as targeting groups and have affinity for asialoglycoprotein receptors may be selected from the group consisting of galactose, galactosamine, N-formyl-galactosamine, N-acetyl-galactosamine, N-propionyl-galactosamine, N-n-butyryl-galactosamine, N-isobutyryl-galactosamine, etc.

In some embodiments, the targeting ligand in the present disclosure comprises N-acetyl-galactosamine as a targeting group:

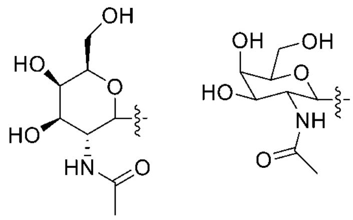

(H-2) (H-2) .

In some embodiments, the targeting ligand comprises three terminal galactosamines or galactosamine derivatives (such as N-acetyl-galactosamine), and each has affinity for asialoglycoprotein receptors. In some embodiments, the targeting ligand comprises three terminal N-acetyl-galactosamines (GalNAc or NAG) as targeting groups.

In some embodiments, the targeting ligand comprises four terminal galactosamines or galactosamine derivatives (such as N-acetyl-galactosamine), and each has affinity for asialoglycoprotein receptors. In some embodiments, the targeting ligand comprises four terminal N-acetyl-galactosamines (GalNAc or NAG) as targeting groups.

Terms commonly used in the art when referring to three terminal N-acetyl-galactosamines include tri-antennary, tri-valent, and trimer.

Terms commonly used in the art when referring to four terminal N-acetyl-galactosamines include tetra-antennary, tetra-valent, and tetramer.

In some embodiments, the targeting ligand provided by the present disclosure is a compound as shown below or a pharmaceutically acceptable salt thereof:

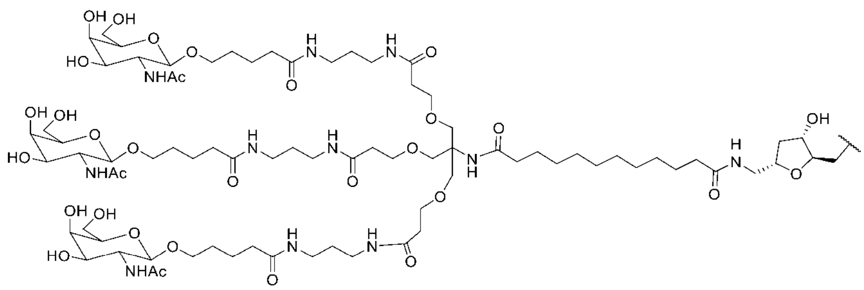

or

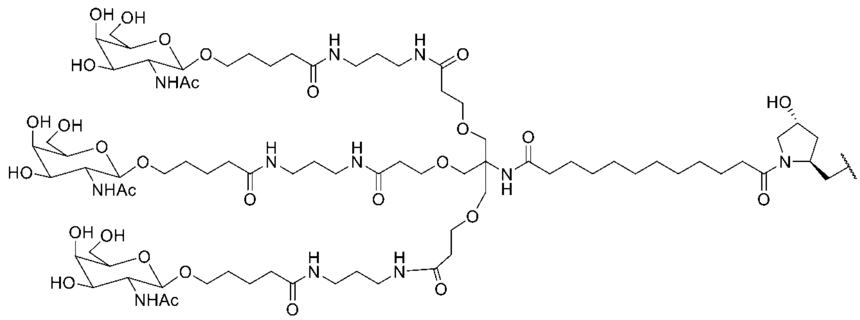

.

In some embodiments, the N-acetyl-galactosamine moiety in the above targeting ligands may be replaced with N-trifluoroacetylgalactosamine, N-propionylgalactosamine, N-n-butyrylgalactosamine, or N-isobutyrylgalactosamine.

In some embodiments, in the RNAi agent described in the present disclosure, the sense strand comprises or is selected from the group consisting of the nucleotide sequence set forth in any one of SEQ ID NO: 21 to SEQ ID NO: 26, and/or the antisense strand comprises or is selected from the group consisting of the nucleotide sequence set forth in any one of SEQ ID NO: 47 to SEQ ID NO: 52.

In some embodiments, the RNAi agent described in the present disclosure comprises or is selected from the group consisting of any one of the following groups:

group 1): a sense strand set forth in SEQ ID NO: 21 and an antisense strand set forth in SEQ ID NO: 47;
group 2): a sense strand set forth in SEQ ID NO: 22 and an antisense strand set forth in SEQ ID NO: 48;
group 3): a sense strand set forth in SEQ ID NO: 23 and an antisense strand set forth in SEQ ID NO: 49;

group 4): a sense strand set forth in SEQ ID NO: 24 and an antisense strand set forth in SEQ ID NO: 50;
group 5): a sense strand set forth in SEQ ID NO: 25 and an antisense strand set forth in SEQ ID NO: 51; and
group 6): a sense strand set forth in SEQ ID NO: 26 and an antisense strand set forth in SEQ ID NO: 52.

In another aspect, the present disclosure provides a pharmaceutical composition comprising the RNAi agent described in the present disclosure, and one or more pharmaceutically acceptable excipients; for example, non-limiting examples of excipients include vehicles, carriers, diluents, and/or delivery polymers.

Various drug delivery systems are known and can be used for the RNAi agent of the present disclosure, e.g., encapsulation in liposomes, microparticles, microcapsules, recombinant cells capable of expressing the RNAi agent, receptor-mediated endocytosis, and construction of a nucleic acid as part of a retroviral vector or other vectors.

In another aspect, the present disclosure provides use of the RNAi agent or the pharmaceutical composition in the manufacture of a medicament for treating a disease in a subject; in some embodiments, the disease is selected from the group consisting of pulmonary diseases.

In another aspect, the present disclosure provides a method for treating a disease in a subject, comprising administering to the subject the RNAi agent or the pharmaceutical composition described in the present disclosure.

In another aspect, the present disclosure provides a method for inhibiting mRNA expression *in vivo* in a subject, and the method comprises administering to the subject the RNAi agent or the pharmaceutical composition of the present disclosure.

In another aspect, the present disclosure provides a method for delivering an RNAi agent inhibiting the expression and/or replication of a target gene to the lungs *in vivo,* and the method comprises administering to a subject the RNAi agent or the pharmaceutical composition described in the present disclosure.

In some embodiments, the target gene is the receptor for advanced glycosylation end-products (RAGE or AGER or "advanced glycosylation end-product specific receptor").

The RNAi agent or the pharmaceutical composition and the methods described in the present disclosure can reduce the level of a target mRNA in a cell, a cell population, a cell population, a tissue, or a subject, comprising administering to the subject a therapeutically effective amount of the RNAi agent or the pharmaceutical composition described in the present disclosure. The RNAi agent is linked to a targeting ligand, thereby inhibiting the expression of the target mRNA in the subject.

In some embodiments, the subject has been previously identified as having pathological up-regulation of the target gene in the targeted cell or tissue.

The subject described in the present disclosure refers to a subject diagnosed with (or suspected to have or susceptible to) a disease or disorder. Without being bound by a specific theory, it is anticipated that the subject would benefit from reducing or inhibiting the expression of the target mRNA.

Delivery may be accomplished by topical administration (e.g., direct injection, implantation, or topical application), systemic administration, or through subcutaneous, intravenous, intraperitoneal, or parenteral routes, including intracranial (e.g., intraventricular, intraparenchymal, and intrathecal), intramuscular, transdermal, airway (aerosol), nasal, oral, rectal, or topical (including buccal and sublingual) administration. In an optional embodiment, the pharmaceutical composition provided by the present disclosure may be administered by injection, e.g., intravenous, intramuscular, intradermal, subcutaneous, intraduodenal, or intraperitoneal injection.

In an optional embodiment, the RNAi agent described in the present disclosure can be packaged in a kit.

In some embodiments, the pharmaceutical composition may further comprise a pharmaceutically acceptable auxiliary material and/or adjuvant, and the auxiliary material may be one or more of the various formulations or compounds conventionally used in the art. For example, the pharmaceutically acceptable auxiliary material may include at least one of a pH buffer, a protective agent, and an osmotic pressure regulator.

In some embodiments, a unit dose of the pharmaceutical composition is 0.001 mg-1000 mg.

In certain embodiments, the pharmaceutical composition comprises 0.01-99.99% of the RNAi agent of the present disclosure based on the total weight of the pharmaceutical composition. In certain embodiments, the pharmaceutical composition comprises 0.1-99.9% of the RNAi agent of the present disclosure. In certain embodiments, the pharmaceutical composition comprises 0.5%-99.5% of the RNAi agent of the present disclosure. In certain embodiments, the pharmaceutical composition comprises 1%-99% of the RNAi agent of the present disclosure. In certain embodiments, the pharmaceutical composition comprises 2%-98% of the RNAi agent of the present disclosure.

In certain embodiments, the pharmaceutical composition comprises 0.01%-99.99% of a pharmaceutically acceptable excipient based on the total weight of the pharmaceutical composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

In some embodiments, when the RNAi agent or the pharmaceutical composition of the present disclosure is in

contact with a target gene-expressing cell, the RNAi agent or the pharmaceutical composition of the present disclosure inhibits the expression of the target gene by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the RNAi agent or the pharmaceutical composition of the present disclosure is in contact with a target gene-expressing cell, the RNAi agent or the pharmaceutical composition of the present disclosure results in a remaining expression percentage of the target gene mRNA of no greater than 99%, no greater than 95%, no greater than 90%, no greater than 85%, no greater than 80%, no greater than 75%, no greater than 70%, no greater than 65%, no greater than 60%, no greater than 55%, no greater than 50%, no greater than 45%, no greater than 40%, no greater than 35%, no greater than 30%, no greater than 25%, no greater than 20%, no greater than 15%, or no greater than 10%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the RNAi agent or the pharmaceutical composition of the present disclosure is in contact with a target gene-expressing cell, the RNAi agent, while retaining on-target activity, reduces off-target activity by at least 20%, at least 25%, at least 30%, at least 35%, at least 40%, at least 45%, at least 50%, at least 55%, at least 60%, at least 65%, at least 70%, or at least 75%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the RNAi agent or the pharmaceutical composition of the present disclosure is in contact with a target gene-expressing cell, the RNAi agent, while reducing on-target activity by at most 20%, at most 19%, at most 15%, at most 10%, at most 5%, or more than 1%, reduces off-target activity by at least 20%, at least 25%, at least 30%, at least 35%, at least 40%, at least 45%, at least 50%, at least 55%, at least 60%, at least 65%, at least 70%, or at least 75%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the RNAi agent or the pharmaceutical composition of the present disclosure is in contact with a target gene-expressing cell, the RNAi agent, while increasing on-target activity by at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, or at least 80%, reduces off-target activity by at least 20%, at least 25%, at least 30%, at least 35%, at least 40%, at least 45%, at least 50%, at least 55%, at least 60%, at least 65%, at least 70%, or at least 75%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

The present disclosure further provides a cell comprising the RNAi agent described in the present disclosure.

The present disclosure further provides a kit comprising the RNAi agent or the pharmaceutical composition described in the present disclosure.

The present disclosure further provides a method for silencing a target gene or mRNA of the target gene in a cell, and the method comprises a step of introducing into the cell the RNAi agent and/or the pharmaceutical composition according to the present disclosure.

The present disclosure further provides a method for silencing a target gene or mRNA of the target gene in a cell *in vivo* or *in vitro,* and the method comprises a step of introducing into the cell the RNAi agent and/or the pharmaceutical composition according to the present disclosure.

The present disclosure further provides a method for inhibiting the expression of a target gene or mRNA of the target gene, and the method comprises administering to a subject in need thereof an effective amount or effective dose of the RNAi agent and/or the pharmaceutical composition according to the present disclosure.

In some embodiments, administration is performed through administration modes including intramuscular, intrabronchial, intrapleural, intraperitoneal, intraarterial, lymphatic, intravenous, subcutaneous, cerebrospinal, or combinations thereof.

In some embodiments, the effective amount or effective dose of the RNAi agent and/or the pharmaceutical composition is about 0.001 mg/kg body weight to about 200 mg/kg body weight, about 0.01 mg/kg body weight to about 100 mg/kg body weight, or about 0.5 mg/kg body weight to about 50 mg/kg body weight.

In the present disclosure, RAGE should be interpreted broadly to refer to the RAGE gene itself and various forms of expression products thereof in various stages, such as but not limited to molecules produced by the gene during amplification, replication, transcription, splicing, processing, translation, and modification, e.g., cDNA, mRNA, precursor

proteins, mature proteins, natural variants, modified forms, and fragments thereof.

In the present disclosure, RAGE is human (*Homo sapiens*) RAGE.

In some embodiments, the target gene is the receptor for advanced glycosylation end-products (RAGE or AGER or "advanced glycosylation end-product specific receptor"). As a specific example, the target gene is the mRNA of RAGE.

The present disclosure provides the RNAi agent and/or the pharmaceutical composition.

The RNAi agent or the pharmaceutical composition is capable of inhibiting RAGE gene expression and/or replication.

The present disclosure provides the RNAi agent and/or the pharmaceutical composition for use in the treatment and/or prevention of a disease associated with RAGE gene expression in a subject. In some embodiments, the disease is selected from the group consisting of diseases, disorders, and symptoms at least partially mediated by RAGE activity and/or AGER gene expression. In some embodiments, the disease is selected from the group consisting of a pulmonary disease. In some embodiments, the pulmonary disease is selected from the group consisting of asthma, acute respiratory distress syndrome, idiopathic pulmonary fibrosis, chronic obstructive pulmonary disease, cystic fibrosis, and pneumonia.

The present disclosure provides a method for treating and/or preventing a disease associated with RAGE gene expression in a subject, comprising administering to the subject an effective amount or effective dose of the RNAi agent or the pharmaceutical composition described in the present disclosure. In some embodiments, the disease is selected from the group consisting of pulmonary diseases associated with the RAGE gene. In some embodiments, the disease is selected from the group consisting of a pulmonary disease; in some embodiments, the pulmonary disease is selected from the group consisting of asthma, acute respiratory distress syndrome, idiopathic pulmonary fibrosis, chronic obstructive pulmonary disease, cystic fibrosis, and pneumonia.

The present disclosure provides use of the RNAi agent and/or the pharmaceutical composition in the manufacture of a medicament for treating and/or preventing a disease associated with RAGE gene expression.

The RNAi agent and/or the pharmaceutical composition provided by the present disclosure can be used for treating a pulmonary disease. In some embodiments, the pulmonary disease is selected from the group consisting of pulmonary diseases associated with the RAGE gene. In some embodiments, the pulmonary disease is selected from the group consisting of asthma, acute respiratory distress syndrome, idiopathic pulmonary fibrosis, chronic obstructive pulmonary disease, cystic fibrosis, and pneumonia.

The present disclosure provides use of the RNAi agent and/or the pharmaceutical composition in the manufacture of a medicament for inhibiting RAGE expression.

The present disclosure provides a method for inhibiting RAGE expression, comprising administering to a subject an effective amount or effective dose of the RNAi agent and/or the pharmaceutical composition of the present disclosure.

The present disclosure provides a method for treating and/or preventing a disease, comprising administering to a subject an effective amount or effective dose of the RNAi agent and/or the pharmaceutical composition of the present disclosure. In some embodiments, the disease is selected from the group consisting of pulmonary diseases associated with the RAGE gene. In some embodiments, the disease is selected from the group consisting of a pulmonary disease. In some embodiments, the pulmonary disease is selected from the group consisting of asthma, acute respiratory distress syndrome, idiopathic pulmonary fibrosis, chronic obstructive pulmonary disease, cystic fibrosis, and pneumonia.

The present disclosure provides use of the RNAi agent and/or the pharmaceutical composition in the manufacture of a medicament for treating and/or preventing a disease. In some embodiments, the disease is selected from the group consisting of pulmonary diseases associated with the RAGE gene. In some embodiments, the disease is selected from the group consisting of pulmonary diseases (asthma, acute respiratory distress syndrome, idiopathic pulmonary fibrosis, chronic obstructive pulmonary disease, cystic fibrosis, and pneumonia).

The present disclosure provides a method for delivering an RNAi agent inhibiting RAGE expression and/or replication *in vivo,* and the method comprises administering to a subject the RNAi agent and/or the pharmaceutical composition of the present disclosure.

The present disclosure further provides a method for preparing an RNAi agent, comprising: synthesizing the RNAi agent described in the present disclosure.

The pharmaceutically acceptable salts of the compounds described in the present disclosure are selected from the group consisting of inorganic salts and organic salts. The compounds of the present disclosure can react with acidic or basic substances to form corresponding salts.

In another aspect, where the configuration is not specified, the compounds of the present disclosure may have particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which fall within the scope of the present disclosure. Additional asymmetric carbon atoms may be present in substituents such as an alkyl group. All such isomers and mixtures thereof fall within the scope of the present disclosure.

In addition, when the configuration is not specified, the compounds and intermediates described in the present disclosure can also be present in different tautomeric forms, and all such forms fall within the scope of the present

disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol, imine-enamine, and lactam-lactim isomerization.

The compounds of the present disclosure may be asymmetric, for example, having one or more stereoisomers. Unless otherwise specified, all stereoisomers are included, for example, enantiomers and diastereomers. The compounds of the present disclosure containing asymmetric carbon atoms may be separated in an optically active pure form or in a racemic form. The optically active pure form may be isolated from a racemic mixture or synthesized using chiral starting materials or chiral reagents.

Optically active (R)- and (S)-isomers, and D- and L-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines).

Where the configurations are not specified, in the chemical structures of the compounds of the present disclosure, the bond "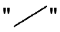" indicates an unspecified configuration; that is, if chiral isomers exist in the chemical structures, the bond "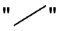" may be "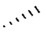" or "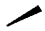", or includes both the configurations "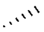" and "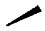" simultaneously. Although all of the structural formulas of the present disclosure are drawn as certain isomeric forms for the sake of simplicity, the present disclosure may include all isomers, such as tautomers, rotamers, geometric isomers, diastereomers, racemates, and enantiomers. In the chemical structures of the compounds of the present disclosure, the bond "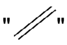" does not specify a configuration; that is, the configuration of the bond "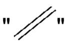" may be an E configuration or a Z configuration, or includes both the E configuration and the Z configuration simultaneously.

WO2022028462A, WO2023274395A, and WO2023208023A are incorporated in the present disclosure by reference in their entirety.

## Terms and Definitions

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains.

As used herein, "RNAi agent" (also referred to as "RNAi trigger agent") means an RNA or RNA-like (e.g., chemically modified RNA) oligonucleotide molecule that is capable of degrading or inhibiting (e.g., degrading or inhibiting under appropriate conditions) a target mRNA in a sequence-specific manner. The RNAi agent used herein may act through an RNA interference mechanism (i.e., inducing RNA interference through interaction with the RNA interference pathway machinery (RNA-induced silencing complex or RISC) of mammalian cells) or through any alternative mechanism or pathway. Although the term RNAi agent as used herein is believed to act primarily through an RNA interference mechanism, the disclosed RNAi agent is not bound or limited by any particular pathway or mechanism of action. The RNAi agent disclosed herein comprises a sense strand and an antisense strand, and includes, but is not limited to, short (or small) interfering RNAs (siRNAs). The antisense strand of the RNAi agent described herein is at least partially complementary to the targeted mRNA. The RNAi agent may comprise one or more modified nucleotides and/or one or more phosphodiester groups with a modification group.

Unless otherwise specified, in the context of the present disclosure, the terms "receptor for advanced glycosylation end-products", "RAGE", "AGER", and "advanced glycosylation end-product specific receptor" are used interchangeably in the present disclosure. RAGE includes, but is not limited to, human RAGE, cynomolgus monkey RAGE, mouse RAGE, and rat RAGE, and their amino acids, complete coding sequences, and mRNA sequences are readily accessible using publicly available databases, e.g., GenBank, UniProt, OMIM, and the Macaca genome project website.

The term "RAGE" also refers to naturally occurring DNA sequence variations of the RAGE gene, such as a single nucleotide polymorphism (SNP) in the RAGE gene. Exemplary SNPs may be found in the dbSNP database.

The term "target sequence" refers to a contiguous portion of the nucleotide sequence of an mRNA molecule formed during the transcription of RAGE, including mRNA that is a product of RNA processing of a primary transcription product. In one embodiment, the target sequence is within the protein-coding region of RAGE.

The term "pulmonary diseases associated with the RAGE gene" includes any disease or disorder associated with reduced RAGE expression and/or activity. Typical " pulmonary diseases associated with the RAGE gene" include

pulmonary diseases (asthma, acute respiratory distress syndrome, idiopathic pulmonary fibrosis, chronic obstructive pulmonary disease, cystic fibrosis, and pneumonia).

The term "αvβ6" is an epithelium-specific integrin that is known as a receptor for ECM proteins and TGF-β latency-associated peptides (LAPs) and is expressed in various cells and tissues. The integrin αvβ6 is known to be significantly up-regulated in damaged lung epithelium.

As used herein, in the context of RNA-mediated gene silencing, the sense strand (also referred to as SS or SS strand) refers to a strand comprising a sequence identical or substantially identical (a sequence differing by no more than 3 nucleotides) to a target mRNA sequence; the antisense strand (also referred to as AS or AS strand) refers to a strand comprising a sequence complementary or partially complementary (a sequence with no more than 3 nucleotide mismatches) to a target mRNA sequence.

In the context describing the sense strand of the RNAi agent described herein, the term "a sequence differing from any one of the nucleotide sequences of SEQ ID NO: 1 to SEQ ID NO: 8 by no more than 3 nucleotides and comprises at least 15 contiguous nucleotides" is intended to mean that the sense strand of the RNAi agent described herein comprises at least 15 contiguous nucleotides of any one of the sense strands of SEQ ID NO: 1 to SEQ ID NO: 8 or a sequence differing from at least 15 contiguous nucleotides of any one of the sense strands of SEQ ID NO: 1 to SEQ ID NO: 8 by no more than 3 nucleotides (illustratively by no more than 2 nucleotides; illustratively by one nucleotide). Illustratively, the sense strand of the RNAi agent described herein comprises at least 16 contiguous nucleotides of any one of the sense strands of SEQ ID NO: 1 to SEQ ID NO: 8 or a sequence differing from at least 16 contiguous nucleotides of any one of the sense strands of SEQ ID NO: 1 to SEQ ID NO: 8 by no more than 3 nucleotides (illustratively by no more than 2 nucleotides; illustratively by one nucleotide).

In the context describing the antisense strand of the RNAi agent described herein, the term "a sequence differing from any one of the antisense strands of SEQ ID NO: 27 to SEQ ID NO: 34 by no more than 3 nucleotides and comprises at least 15 contiguous nucleotides" is intended to mean that the antisense strand of the RNAi agent described herein comprises at least 15 contiguous nucleotides of any one of the antisense strands of SEQ ID NO: 27 to SEQ ID NO: 34 or a sequence differing from at least 15 contiguous nucleotides of any one of the antisense strands of SEQ ID NO: 27 to SEQ ID NO: 34 by no more than 3 nucleotides (illustratively by no more than 2 nucleotides; illustratively by one nucleotide).

In the present disclosure, the "5' region", "5' end", and "5' terminus" of the sense or antisense strand are used interchangeably. For example, the nucleotides at positions 2 to 8 of the 5' region of the antisense strand may also be replaced with the nucleotides at positions 2 to 8 of the 5' end of the antisense strand. Likewise, the "3' region", "3' terminus", and "3' end" of the sense or antisense strand are also used interchangeably.

Unless otherwise specified, in the context of the present disclosure, "G", "C", "A", "T", and "U" represent nucleotides, and comprise the bases of guanine, cytosine, adenine, thymidine, and uracil, respectively. The lowercase letter m means that the upstream nucleotide adjacent to the letter m is a methoxy-modified nucleotide; the lowercase letter f means that the upstream nucleotide adjacent to the letter f is a fluoro-modified nucleotide; the lowercase letter s means that the two nucleotides adjacent to the letter s are linked by a phosphorothioate diester group.

As used in the present disclosure, the term "2'-fluoro-modified nucleotide" refers to a nucleotide in which the hydroxy group at the 2' position of the ribosyl group of the nucleotide is substituted with fluorine.

As used in the present disclosure, the term "2'-methoxy-modified nucleotide" refers to a nucleotide in which the 2'-hydroxy group of the ribosyl group is substituted with a methoxy group.

As used herein, the terms "complementary" and "reverse complementary" are used interchangeably and have the meaning well-known to those skilled in the art; that is, in a double-stranded nucleic acid molecule, the bases of one strand are paired with the bases of the other strand in a complementary manner. In DNA, the purine base adenine is always paired with the pyrimidine base thymine (or uracil in RNA), and the purine base guanine is always paired with the pyrimidine base cytosine. Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases at the corresponding positions are not paired in a complementary manner.

As used herein, the term "inhibit" is used interchangeably with "decrease", "silence", "down-regulate", "repress", and other similar terms, and includes any level of inhibition. Inhibition can be assessed in terms of a decrease in the absolute or relative level of one or more of these variables relative to a control level. The control level can be any type of control level used in the art, such as a pre-dose baseline level or a level determined from an untreated or control (e.g., buffer-only control or inert agent control) treated subject, cell, or sample. For example, the remaining mRNA expression level can be used to characterize the degree of inhibition of target gene expression by the RNAi agent; for example, the remaining expression level of mRNA is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition rate of

target gene expression can be measured using a Dual-Glo® Luciferase Assay System: the Firefly chemiluminescence value (Fir) and the Renilla chemiluminescence value (Ren) are each read, and the relative value Ratio = Ren/Fir is calculated. In the present disclosure, the ratio of the remaining mRNA expression level (or residual activity%) = Ratio (RNAi agent-treated group)/Ratio (RNAi agent-free control group), and the inhibition rate (%) = 100% - remaining mRNA expression level (%).

Unless otherwise specified, the "compound", "ligand", "nucleic acid-ligand conjugate", "nucleic acid", "conjugate", "chemical modification", "targeting ligand", "RNAi agent", "siRNA", and "dsRNA" of the present disclosure can each independently be present in the form of a salt, a mixed salt, or a non-salt (e.g., a free acid or free base). When being present in the form of a salt or mixed salts, it may be a pharmaceutically acceptable salt. The term "pharmaceutically acceptable salt" includes pharmaceutically acceptable acid addition salts and pharmaceutically acceptable base addition salts.

"Pharmaceutically acceptable acid addition salt" refers to salts that are capable of retaining the biological effectiveness of free bases without having any undesirable effects and that are formed with inorganic or organic acids. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-aminosalicylates, napadisylates, etc. These salts can be prepared using methods known in the art.

"Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. The inorganic salts in some embodiments are ammonium salts, sodium salts, potassium salts, calcium salts, and magnesium salts, and the inorganic salts in some embodiments are sodium salts. Salts derived from organic bases include, but are not limited to, salts of the following: primary, secondary, and tertiary amines, substituted amines including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. The organic bases in some embodiments include isopropylamine, diethylamine, ethanolamine, trimethylamine, dicyclohexylamine, choline, and caffeine. These salts can be prepared using methods known in the art.

"Effective amount" or "effective dose" refers to the amount of an RNAi agent, a compound, or a pharmaceutical composition necessary to obtain any one or more beneficial or desired prophylactic or therapeutic results. For prophylactic use, the beneficial or desired results include elimination or reduction of risk, reduction of severity, or delay of the onset of a disorder, including the biochemistry, histology, and/or behavioral symptoms of the disorder, complications thereof, and intermediate pathological phenotypes that appear during the progression of the disorder. For therapeutic applications, the beneficial or desired results include clinical results, such as reducing the incidence of various disorders related to the target gene, target mRNA, or target protein of the present disclosure or ameliorating one or more symptoms of the disorders, reducing the dose of other agents required to treat the disorders, enhancing the therapeutic effect of another agent, and/or delaying the progression of disorders related to the target gene, target mRNA, or target protein of the present disclosure in patients.

As used herein, "patient", "subject", and "individual" are used interchangeably and include human or non-human animals, e.g., mammals, e.g., humans or monkeys.

The RNAi agent provided by the present disclosure can be obtained using a preparation method conventional in the art (e.g., solid-phase synthesis and liquid-phase synthesis). Solid-phase synthesis has been commercially available as a customization service. A modified nucleotide group can be introduced into the RNAi agent described in the present disclosure using a nucleoside monomer with a corresponding modification. Methods of preparing a nucleoside monomer with a corresponding modification and introducing a modified nucleotide group into an RNAi agent are also well known to those skilled in the art.

The term "chemical modification" or "modification" includes all changes made to a nucleotide by chemical means, such as the addition or removal of a chemical moiety, or the substitution of one chemical moiety for another.

The term "base" encompasses any known DNA and RNA bases and base analogs such as purines or pyrimidines, and also includes the natural compounds adenine, thymine, guanine, cytosine, uracil, inosine, and natural analogs.

The terms "blunt" and "blunt-ended" are used interchangeably and mean that there are no unpaired nucleotides

or nucleotide analogs at a given end of an RNAi agent, i.e., no nucleotide overhang. In most cases, an RNAi agent whose ends are both blunt-ended will be double-stranded over its entire length.

The terms "about" and "approximately" mean that a numerical value is within an acceptable margin of error for the specific value determined by those of ordinary skill in the art, and the numerical value depends in part on how the measurement is performed (i.e., the limitations of the measurement system). For example, "about" may mean a standard deviation within 1 or greater than 1. Alternatively, "about" or "substantially comprise" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes an embodiment of the given number. Unless otherwise specified, when a specific value is provided in the present application and claims, the meaning of "about" or "substantially comprise" should be assumed to be within an acceptable margin of error for that specific value.

Unless otherwise specified, "optionally" or "optional" means that the event or circumstance subsequently described may, but does not necessarily, occur, and this description includes instances where the event or circumstance occurs or does not occur. For example, "optionally, $R_1$ and $R_2$ are directly linked to form a ring" means that $R_1$ and $R_2$ being directly linked to form a ring may occur, but does not necessarily exist, and this description includes an instance where $R_1$ and $R_2$ are directly linked to form a ring and an instance where $R_1$ and $R_2$ do not form a ring.

In the chemical structural formulas of the present disclosure, "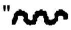" or 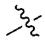 may be linked to any group or groups in accordance with the scope of the invention described herein.

The term "link", when referring to a relationship between two molecules, means that the two molecules are linked by a covalent bond or that the two molecules are associated via a non-covalent bond (e.g., a hydrogen bond or an ionic bond), and includes direct linkage and indirect linkage.

The term "directly linked" means that a first compound or group is linked to a second compound or group without any atom or group of atoms interposed between.

The term "indirectly linked" means that a first compound or group is linked to a second compound or group by an intermediate group, a compound, or a molecule (e.g., a linking group).

The term "substituted" means that any one or more hydrogen atoms on the specified atom (usually a carbon, oxygen, or nitrogen atom) are replaced with any group as defined herein, provided that the normal valency of the specified atom is not exceeded and that the substitution results in a stable compound. Non-limiting examples of substituents include C1-C6 alkyl, C2-C6 alkenyl, C2-C6 alkynyl, cyano, hydroxy, oxo, carboxyl, cycloalkyl, cycloalkenyl, heterocyclyl, heteroaryl, aryl, ketone, alkoxycarbonyl, aryloxycarbonyl, heteroaryloxycarbonyl, or halogen (e.g., F, Cl, Br, or I). When the substituent is a ketone or oxo (i.e., =O), two (2) hydrogens on the atom are replaced.

"Substituted with one or more..." refers to substitution with a single substituent or multiple substituents. In the case of substitution with multiple substituents, there may be a plurality of identical substituents, or one or a group of a plurality of different substituents.

## DETAILED DESCRIPTION

The present disclosure is further described below with reference to examples, but these examples are not intended to limit the scope of the present disclosure. In the examples of the present disclosure, experimental methods without specific conditions specified were generally conducted under conventional conditions, such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific sources indicated were commercially available conventional reagents.

### Example 1. Design of Human RAGE RNAi Agents

With the human RAGE gene (NM-001136.5) as a target gene, RNAi agents of 19/21nt were designed in accordance with the general rules for active RNAi agents. The sequences of modified or unmodified sense and antisense strands are shown in Tables 1, 2, and 3.

Table 1. The modified or unmodified sense and antisense strands of human RAGE RNAi agents

| Double strand No. | SEQ ID NO: | SS strand (5'-3') | SEQ ID NO: | AS strand (5'-3') |
|---|---|---|---|---|
| TJR103569 | 1 | CUGUCAGCAUCAGCAUCAA | 27 | UUGAUGCUGAUGCUGACAGCA |

(continued)

| Double strand No. | SEQ ID NO: | SS strand (5'-3') | SEQ ID NO: | AS strand (5'-3') |
|---|---|---|---|---|
| TJR103570 | 2 | GACCAACUCUCUCCU GUAA | 28 | UUACAGGAGAGAGUUGGU CUG |
| TJR103571 | 3 | AGAGGAGGAGCGUG CAGAA | 29 | UUCUGCACGCUCCUCCUCU UC |
| TJR103572 | 4 | CAGUCGGAGCUAAU GGUGA | 30 | UCACCAUUAGCUCCGACUG CA |
| TJR103573 | 5 | CUUUUCUUUUUCCC UUGAA | 31 | UUCAAGGGAAAAAGAAAA GGG |
| TJR103574 | 6 | CCUUUUCUUUUUCCC UUGA | 32 | UCAAGGGAAAAAGAAAAG GGA |
| TJR103578 | 7 | CUGUCAGCAUCAGC AUCAU | 33 | AUGAUGCUGAUGCUGACA GCA |
| TJR103781 | 8 | GACCAACUCUCUCCU GUAU | 34 | AUACAGGAGAGAGUUGGU CUG |
| TJR103575 | 275 | CGUGCUGUCAGCAU CAGCA | 276 | UGCUGAUGCUGACAGCACG GC |
| TJR103577 | 277 | UGUCAGCAUCAGCA UCAUC | 278 | GAUGAUGCUGAUGCUGAC AGC |
| TJR101314 | 9 | CmsUmsGmUmCmAmG fCfAfUmCmAmGmCmA mUmCmAmAm | 35 | VPUmsUfsGmAmUmGfCmUm GmAmUmGfCmUfGmAfCmA mGmsCmsAm |
| TJR101321 | 10 | GmsAmsCmCmAmAmC fUfCfUmCmUmCmCmU mGmUmAmAm | 36 | VPUmsUfsAmCmAmGfGmAm GmAmGmAfGmUfUmGfGmU mCmsUmsGm |
| TJR101317 | 11 | AmsGmsAmGmGmAmG fGfAfGmCmGmUmGm CmAmGmAmAm | 37 | VPUmsUfsCmUmGmCfAmCm GmCmUmCfCmUfCmCfUmCm UmsUmsCm |
| TJR101303 | 12 | CmsAmsGmUmCmGmG fAfGfCmUmAmAmUm GmGmUmGmAm | 38 | VPUmsCfsAmCmCmAfUmUm AmGmCmUfCmCfGmAfCmUm GmsCmsAm |
| TJR101319 | 13 | CmsUmsUmUmUmCmU fUfUfUmUmCmCmCmU mUmGmAmAm | 39 | VPUmsUfsCmAmAmGfGmGm AmAmAmAfAmGfAmAfAmA mGmsGmsGm |
| TJR101318 | 14 | CmsCmsUmUmUmUmC fUfUfUmUmUmCmCmC mUmUmGmAm | 40 | VPUmsCfsAmAmGmGfGmAm AmAmAmAfGmAfAmAfAmG mGmsGmsAm |
|  | 15 | CmsUmsGmUmCmAmG fCfAfUmCmAmGmCmA m UmCmsAmsAm | 41 | UmsUfsGmAfUmGfCmUmGm AfUmGfCmUfGmAfCmAfGms CmsAm |

(continued)

| Double strand No. | SEQ ID NO: | SS strand (5'-3') | SEQ ID NO: | AS strand (5'-3') |
|---|---|---|---|---|
| | 16 | GmsAmsCmCmAmAmCfUfCfUmCmUmCmCmUm GmUmsAmsAm | 42 | UmsUfsAmCfAmGfGmAmGmAfGmAfGmUfUmGfGmUfCmsUmsGm |
| | 17 | AmsGmsAmGmGmAmGfGfAfGmCmGmUmGmCm AmGmsAmsAm | 43 | UmsUfsCmUfGmCfAmCmGmCfUmCfCmUfCmCfUmCfUmsUmsCm |
| | 18 | CmsAmsGmUmCmGmGfAfGfCmUmAmAmUmGm GmUmsGmsAm | 44 | UmsCfsAmCfCmAfUmUmAmGfCmUfCmCfGmAfCmUfGmsCmsAm |
| | 19 | CmsUmsUmUmUmCmUfUfUfUmUmCmCmCmUm UmGmsAmsAm | 45 | UmsUfsCmAfAmGfGmGmAmAfAmAfAmGfAmAfAmAfGmsGmsGm |
| | 20 | CmsCmsUmUmUmUmCfUfUfUmUmUmCmCmCm UmUmsGmsAm | 46 | UmsCfsAmAfGmGfGmAmAmAfAmAfGmAfAmAfAmGmGmsGmsAm |
| TJR103633 -TJ-16 | 21 | **I-14**CmsUmsGmUmCmAmGfCfAfUmCmAmGmCmAmUmCmsAmsAm | 47 | UmsUfsGm AfUmGf CmUmGm AfUmGf CmUfGm AfCmAf GmsCmsAm |
| TJR103634 -TJ-16 | 22 | **I-14**GmsAmsCm CmAmAm CfUfCf UmCmUm CmCmUm GmUmsAms Am | 48 | UmsUfsAm CfAmGf GmAmGm AfGmAf GmUfUm GfGmUf CmsUmsGm |
| TJR103635 -TJ-16 | 23 | **I-14**AmsGmsAm GmGmAm GfGfAf GmCmGm UmGmCm AmGmsAms Am | 49 | UmsUfsCm UfGmCf AmCmGm CfUmCf CmUfCm CfUmCf UmsUmsCm |
| TJR103636 -TJ-16 | 24 | **I-14**CmsAmsGm UmCmGm GfAfGf CmUmAm AmUmGm GmUmsGms Am | 50 | UmsCfsAm CfCmAf UmUmAm GfCmUf CmCfGm AfCmUf GmsCmsAm |
| TJR103637 -TJ-16 | 25 | **I-14**CmsUmsUm UmUmCm UfUfUf UmUmCm CmCmUm UmGmsAms Am | 51 | UmsUfsCm AfAmGf GmGmAm AfAmAf AmGfAm AfAmAf GmsGmsGm |
| TJR103638 -TJ-16 | 26 | **I-14**CmsCmsUm UmUmUm CfUfUf UmUmUm CmCmCm UmUmsGms Am | 52 | UmsCfsAm AfGmGf GmAmAm AfAmAf GmAfAm AfAmGm GmsGmsAm |

Table 2. The unmodified sense and antisense strands of human RAGE RNAi agents

| SEQ ID NO: | Unmodified SS strand (5'-3') | SEQ ID NO: | Unmodified AS strand (5'-3') |
|---|---|---|---|
| 53 | GGGCAGUAGUAGGUGCUCA | 107 | UGAGCACCUACUACUGCCCCC |
| 54 | GGCAGUAGUAGGUGCUCAA | 108 | UUGAGCACCUACUACUGCCCC |

(continued)

| SEQ ID NO: | Unmodified SS strand (5'-3') | SEQ ID NO: | Unmodified AS strand (5'-3') |
|---|---|---|---|
| 55 | GCAGUAGUAGGUGCUCAAA | 109 | UUUGAGCACCUACUACUGCCC |
| 56 | CAGUAGUAGGUGCUCAAAA | 110 | UUUUGAGCACCUACUACUGCC |
| 57 | CUGUCGGGAUCCAGGAUGA | 111 | UCAUCCUGGAUCCCGACAGCC |
| 58 | CGGUGCCAGGCAAUGAACA | 112 | UGUUCAUUGCCUGGCACCGGA |
| 59 | GCCAGGCAAUGAACAGGAA | 113 | UUCCUGUUCAUUGCCUGGCAC |
| 60 | GGAAUGGAAAGGAGACCAA | 114 | UUGGUCUCCUUUCCAUUCCUG |
| 61 | GGAAAGGAGACCAAGUCCA | 115 | UGGACUUGGUCUCCUUUCCAU |
| 62 | GAAAGGAGACCAAGUCCAA | 116 | UUGGACUUGGUCUCCUUUCCA |
| 63 | AGGAGACCAAGUCCAACUA | 117 | UAGUUGGACUUGGUCUCCUUU |
| 64 | GACCAAGUCCAACUACCGA | 118 | UCGGUAGUUGGACUUGGUCUC |
| 65 | CACGGCUGGUGUUCCCAAU | 119 | AUUGGGAACACCAGCCGUGAG |
| 66 | ACAUGUGUGUCAGAGGGAA | 120 | UUCCCUCUGACACACAUGUCC |
| 67 | GUGUGUCAGAGGGAAGCUA | 121 | UAGCUUCCCUCUGACACACAU |
| 68 | CCCCUGGUGCCUAAUGAGA | 122 | UCUCAUUAGGCACCAGGGGCU |
| 69 | CCCUGGUGCCUAAUGAGAA | 123 | UUCUCAUUAGGCACCAGGGGC |
| 70 | GGGAGUAUCUGUGAAGGAA | 124 | UUCCUUCACAGAUACUCCCUU |
| 71 | CAGGAGACACCCUGAGACA | 125 | UGUCUCAGGGUGUCUCCUGGU |
| 72 | ACACUGCAGUCGGAGCUAA | 126 | UUAGCUCCGACUGCAGUGUGA |
| 73 | CACUGCAGUCGGAGCUAAU | 127 | AUUAGCUCCGACUGCAGUGUG |
| 74 | GCCAGAAGGUGGAGCAGUA | 128 | UACUGCUCCACCUUCUGGCUC |
| 75 | GCAGUAGCUCCUGGUGGAA | 129 | UUCCACCAGGAGCUACUGCUC |
| 76 | GCUCCUGGUGGAACCGUAA | 130 | UUACGGUUCCACCAGGAGCUA |
| 77 | GGUGGAACCGUAACCCUGA | 131 | UCAGGGUUACGGUUCCACCAG |
| 78 | CCGUAACCCUGACCUGUGA | 132 | UCACAGGUCAGGGUUACGGUU |
| 79 | CGUAACCCUGACCUGUGAA | 133 | UUCACAGGUCAGGGUUACGGU |
| 80 | CUGUGCUGAUCCUCCCUGA | 134 | UCAGGGAGGAUCAGCACAGGG |
| 81 | GCUGAUCCUCCCUGAGAUA | 135 | UAUCUCAGGGAGGAUCAGCAC |
| 82 | CAGGAAAGCCGUGCUGUCA | 136 | UGACAGCACGGCUUUCCUGGG |
| 83 | CGUGCUGUCAGCAUCAGCA | 137 | UGCUGAUGCUGACAGCACGGC |
| 84 | GCAUCAUCGAACCAGGCGA | 138 | UCGCCUGGUUCGAUGAUGCUG |
| 85 | GGGGUCAUCUUGUGGCAAA | 139 | UUUGCCACAAGAUGACCCCAA |
| 86 | CUUUUUCCCUUGAACUGUU | 140 | AACAGUUCAAGGGAAAAAGAA |
| 87 | CCAACUCUCUCCUGUAUAA | 141 | UUAUACAGGAGAGAGUUGGUC |
| 88 | GGCUGUCGGGAUCCAGGAU | 142 | AUCCUGGAUCCCGACAGCCGG |
| 89 | GGAAGCCCCUGGUGCCUAA | 143 | UUAGGCACCAGGGGCUUCCCA |
| 90 | GCCUCUGGAGGAGGUCCAA | 144 | UUGGACCUCCUCCAGAGGCAC |
| 91 | GCCCAGCCCUCUCCUCAAA | 145 | UUUGAGGAGAGGGCUGGGCAG |
| 92 | GGAAGAGGAGGAGCGUGCA | 146 | UGCACGCUCCUCCUCUUCCUC |
| 93 | CAACCAGAGCCCCCCACAA | 147 | UUGUGGGGGGCUCUGGUUGUA |

(continued)

| SEQ ID NO: | Unmodified SS strand (5'-3') | SEQ ID NO: | Unmodified AS strand (5'-3') |
|---|---|---|---|
| 94 | CCAGAGCCCCCCACAAUGA | 148 | UCAUUGUGGGGGGCUCUGGUU |
| 95 | CUGGGACAGUGUGGCUCGU | 149 | ACGAGCCACACUGUCCCAGGG |
| 96 | AGUCCAACUACCGAGUCCA | 150 | UGGACUCGGUAGUUGGACUUG |
| 97 | GUCCAACUACCGAGUCCGU | 151 | ACGGACUCGGUAGUUGGACUU |
| 98 | CAUCACAGCCCGGAUUGGA | 152 | UCCAAUCCGGGCUGUGAUGUU |
| 99 | AAGGUGGGGACAUGUGUGU | 153 | ACACACAUGUCCCCACCUUAU |
| 100 | AGGUGGGGACAUGUGUGUA | 154 | UACACACAUGUCCCCACCUUA |
| 101 | GGUGGGGACAUGUGUGUCA | 155 | UGACACACAUGUCCCCACCUU |
| 102 | GGAAGCUACCCUGCAGGGA | 156 | UCCCUGCAGGGUAGCUUCCCU |
| 103 | GAAGCUACCCUGCAGGGAA | 157 | UUCCCUGCAGGGUAGCUUCCC |
| 104 | AAGCUACCCUGCAGGGACU | 158 | AGUCCCUGCAGGGUAGCUUCC |
| 105 | ACCAGGAGACACCCUGAGA | 159 | UCUCAGGGUGUCUCCUGGUCU |
| 106 | CCAGGAGACACCCUGAGAA | 160 | UUCUCAGGGUGUCUCCUGGUC |

Table 3. The modified sense and antisense strands of human RAGE RNAi agents

| Double strand No. | SEQ ID NO: | SS strand (5'-3') | SEQ ID NO: | AS strand (5'-3') |
|---|---|---|---|---|
| TJR101282 | 161 | GmsGmsGmCmAmGmUfAfGfUmAmGmGmUmGmCmUmCmAm | 215 | VPUmsGfsAmGmCmAfCmCmUmAmCmUfAmCfUmGfCmCmCmsCmsCm |
| TJR101283 | 162 | GmsGmsCmAmGmUmAfGfUfAmGmGmUmGmCmUmCmAmAm | 216 | VPUmsUfsGmAmGmCfAmCmCmUmAmCfUmAfCmUfGmCmCmsCmsCm |
| TJR101284 | 163 | GmsCmsAmGmUmAmGfUfAfGmGmUmGmCmUmCmAmAmAm | 217 | VPUmsUfsUmGmAmGfCmAmCmCmUmAfCmUfAmCfUmGmCmsCmsCm |
| TJR101285 | 164 | CmsAmsGmUmAmGmUfAfGfGmUmGmCmUmCmAmAmAmAm | 218 | VPUmsUfsUmUmGmAfGmCmAmCmCmUfAmCfUmAfCmUmGmsCmsCm |
| TJR101286 | 165 | CmsUmsGmUmCmGmGfGfAfUmCmCmAmGmGmAmUmGmAm | 219 | VPUmsCfsAmUmCmCfUmGmGmAmUmCfCmCfGmAfCmAmGmsCmsCm |
| TJR101287 | 166 | CmsGmsGmUmGmCmCfAfGfGmCmAmAmUmGmAmAmCmAm | 220 | VPUmsGfsUmUmCmAfUmUmGmCmCmUfGmGfCmAfCmCmGmsGmsAm |
| TJR101288 | 167 | GmsCmsCmAmGmGmCfAfAfUmGmAmAmCmAmGmGmAmAm | 221 | VPUmsUfsCmCmUmGfUmUmCmAmUmUfGmCfCmUfGmGmCmsAmsCm |
| TJR101289 | 168 | GmsGmsAmAmUmGmGfAfAfAmGmGmAmGmAmCmCmAmAm | 222 | VPUmsUfsGmGmUmCfUmCmCmUmUmUfCmCfAmUfUmCmCmsUmsGm |

(continued)

| Double strand No. | SEQ ID NO: | SS strand (5'-3') | SEQ ID NO: | AS strand (5'-3') |
|---|---|---|---|---|
| TJR101290 | 169 | GmsGmsAmAmAmGmGfAfGfAmCmCmAmAmGmUmCmCmAm | 223 | VPUmsGfsGmAmCmUfUmGmGmUmCmUfCmCfUmUfUmCmCmsAmsUm |
| TJR101291 | 170 | GmsAmsAmAmGmGmAfGfAfCmCmAmAmGmUmCmCmAmAm | 224 | VPUmsUfsGmGmAmCfUmUmGmGmUmCfUmCfCmUfUmUmCmsCmsAm |
| TJR101292 | 171 | AmsGmsGmAmGmAmCfCfAfAmGmUmCmCmAmAmCmUmAm | 225 | VPUmsAfsGmUmUmGfGmAmCmUmUmGfGmUfCmUfCmCmUmsUmsUm |
| TJR101293 | 172 | GmsAmsCmCmAmAmGfUfCfCmAmAmCmUmAmCmCmGmAm | 226 | VPUmsCfsGmGmUmAfGmUmUmGmGmAfCmUfUmGfGmUmCmsUmsCm |
| TJR101294 | 173 | CmsAmsCmGmGmCmUfGfGfUmGmUmUmCmCmCmAmAmAm | 227 | VPUmsUfsUmGmGmGfAmAmCmAmCmCfAmGfCmCfGmUmGmsAmsGm |
| TJR101295 | 174 | AmsCmsAmUmGmUmGfUfGfUmCmAmGmAmGmGmGmAmAm | 228 | VPUmsUfsCmCmCmUfCmUmGmAmCmAfCmAfCmAfUmGmUmsCmsCm |
| TJR101296 | 175 | GmsUmsGmUmGmUmCfAfGfAmGmGmGmAmAmGmCmUmAm | 229 | VPUmsAfsGmCmUmUfCmCmCmUmCmUfGmAfCmAfCmAmCmsAmsUm |
| TJR101297 | 176 | CmsCmsCmCmUmGmGfUfGfCmCmUmAmAmUmGmAmGmAm | 230 | VPUmsCfsUmCmAmUfUmAmGmGmCmAfCmCfAmGfGmGmGmsCmsUm |
| TJR101298 | 177 | CmsCmsCmUmGmGmUfGfCfCmUmAmAmUmGmAmGmAmAm | 231 | VPUmsUfsCmUmCmAfUmUmAmGmGmCfAmCfCmAfGmGmGmsGmsCm |
| TJR101299 | 178 | GmsGmsGmAmGmUmAfUfCfUmGmUmGmAmAmGmGmAmAm | 232 | VPUmsUfsCmCmUmUfCmAmCmAmGmAfUmAfCmUfCmCmCmsUmsUm |
| TJR101300 | 179 | CmsAmsGmGmAmGmAfCfAfCmCmCmUmGmAmGmAmCmAm | 233 | VPUmsGfsUmCmUmCfAmGmGmGmUmGfUmCfUmCfCmUmGmsGmsUm |
| TJR101301 | 180 | AmsCmsAmCmUmGmCfAfGfUmCmGmGmAmGmCmUmAmAm | 234 | VPUmsUfsAmGmCmUfCmCmGmAmCmUfGmCfAmGfUmGmUmsGmsAm |
| TJR101302 | 181 | CmsAmsCmUmGmCmAfGfUfCmGmGmAmGmCmUmAmAmAm | 235 | VPUmsUfsUmAmGmCfUmCmCmGmAmCfUmGfCmAfGmUmGmsUmsGm |
| TJR101304 | 182 | GmsCmsCmAmGmAmAfGfGfUmGmGmAmGmCmAmGmUmAm | 236 | VPUmsAfsCmUmGmCfUmCmCmAmCmCfUmUfCmUfGmGmCmsUmsCm |

(continued)

| Double strand No. | SEQ ID NO: | SS strand (5'-3') | SEQ ID NO: | AS strand (5'-3') |
|---|---|---|---|---|
| TJR101305 | 183 | GmsCmsAmGmUmAmGfCfUfCmCmUmGmGmUmGmGmAmAm | 237 | VPUmsUfsCmCmAmCfCmAmGmGmAmGfCmUfAmCfUmGmCmsUmsCm |
| TJR101306 | 184 | GmsCmsUmCmCmUmGfGfUfGmGmAmAmCmCmGmUmAmAm | 238 | VPUmsUfsAmCmGmGfUmUmCmCmAmCfCmAfGmGfAmGmCmsUmsAm |
| TJR101307 | 185 | GmsGmsUmGmGmAmAfCfCfGmUmAmAmCmCmCmUmGmAm | 239 | VPUmsCfsAmGmGmGfUmUmAmCmGmGfUmUfCmCfAmCmCmsAmsGm |
| TJR101308 | 186 | CmsCmsGmUmAmAmCfCfCfUmGmAmCmCmUmGmUmGmAm | 240 | VPUmsCfsAmCmAmGfGmUmCmAmGmGfGmUfUmAfCmGmGmsUmsUm |
| TJR101309 | 187 | CmsGmsUmAmAmCmCfCfUfGmAmCmCmUmGmUmGmAmAm | 241 | VPUmsUfsCmAmCmAfGmGmUmCmAmGfGmGfUmUfAmCmGmsGmsUm |
| TJR101310 | 188 | CmsUmsGmUmGmCmUfGfAfUmCmCmUmCmCmCmUmGmAm | 242 | VPUmsCfsAmGmGmGfAmGmGmAmUmCfAmGfCmAfCmAmGmsGmsGm |
| TJR101311 | 189 | GmsCmsUmGmAmUmCfCfUfCmCmCmUmGmAmGmAmUmAm | 243 | VPUmsAfsUmCmUmCfAmGmGmGmAmGfGmAfUmCfAmGmCmsAmsCm |
| TJR101312 | 190 | CmsAmsGmGmAmAmAfGfCfCmGmUmGmCmUmGmUmCmAm | 244 | VPUmsGfsAmCmAmGfCmAmCmGmGmCfUmUfUmCfCmUmGmsGmsGm |
| TJR101313 | 191 | CmsGmsUmGmCmUmGfUfCfAmGmCmAmUmCmAmGmCmAm | 245 | VPUmsGfsCmUmGmAfUmGmCmUmGmAfCmAfGmCfAmCmGmsGmsCm |
| TJR101315 | 192 | GmsCmsAmUmCmAmUfCfGfAmAmCmCmAmGmGmCmGmAm | 246 | VPUmsCfsGmCmCmUfGmGmUmUmCmGfAmUfGmAfUmGmCmsUmsGm |
| TJR101316 | 193 | GmsGmsGmGmUmCmAfUfCfUmUmGmUmGmGmCmAmAmAm | 247 | VPUmsUfsUmGmCmCfAmCmAmAmGmAfUmGfAmCfCmCmCmsAmsAm |
| TJR101320 | 194 | CmsUmsUmUmUmUmCfCfCfUmUmGmAmAmCmUmGmUmAm | 248 | VPUmsAfsCmAmGmUfUmCmAmAmGmGfGmAfAmAfAmAmGmsAmsAm |
| TJR101322 | 195 | CmsCmsAmAmCmUmCfUfCfUmCmCmUmGmUmAmUmAmAm | 249 | VPUmsUfsAmUmAmCfAmGmGmAmGmAfGmAfGmUfUmGmGmsUmsCm |
| TJR101323 | 196 | GmsGmsCmUmGmUmCfGfGfGmAmUmCmCmAmGmGmAmAm | 250 | VPUmsUfsCmCmUmGfGmAmUmCmCmCfGmAfCmAfGmCmCmsGmsGm |

(continued)

| Double strand No. | SEQ ID NO: | SS strand (5'-3') | SEQ ID NO: | AS strand (5'-3') |
|---|---|---|---|---|
| TJR101324 | 197 | GmsGmsAmAmGmCmCfCfCfUmGmGmUmGmCmCmUmAmAm | 251 | VPUmsUfsAmGmGmCfAmCmCmAmGmGfGmGfCmUfUmCmCmsCmsAm |
| TJR101325 | 198 | GmsCmsCmUmCmUmGfGfAfGmGmAmGmGmUmCmCmAmAm | 252 | VPUmsUfsGmGmAmCfCmUmCmCmUmCfCmAfGmAfGmGmCmsAmsCm |
| TJR101326 | 199 | GmsCmsCmCmAmGmCfCfCfUmCmUmCmCmUmCmAmAmAm | 253 | VPUmsUfsUmGmAmGfGmAmGmAmGmGfGmCfUmGfGmGmCmsAmsGm |
| TJR101327 | 200 | GmsGmsAmAmGmAmGfGfAfGmGmAmGmCmGmUmGmCmAm | 254 | VPUmsGfsCmAmCmGfCmUmCmCmUmCfCmUfCmUfUmCmCmsUmsCm |
| TJR101328 | 201 | CmsAmsAmCmCmAmGfAfGfCmCmCmCmCmCmAmCmAmAm | 255 | VPUmsUfsGmUmGmGfGmGmGmGmCmUfCmUfGmGfUmUmGmsUmsAm |
| TJR101329 | 202 | CmsCmsAmGmAmGmCfCfCfCmCmCmAmCmAmAmUmGmAm | 256 | VPUmsCfsAmUmUmGfUmGmGmGmGmGfGmCfUmCfUmGmGmsUmsUm |
| TJR101330 | 203 | CmsUmsGmGmGmAmCfAfGfUmGmUmGmGmCmUmCmGmAm | 257 | VPUmsCfsGmAmGmCfCmAmCmAmCmUfGmUfCmCfCmAmGmsGmsGm |
| TJR101331 | 204 | AmsGmsUmCmCmAmAfCfUfAmCmCmGmAmGmUmCmCmAm | 258 | VPUmsGfsGmAmCmUfCmGmGmUmAmGfUmUfGmGfAmCmUmsUmsGm |
| TJR101332 | 205 | GmsUmsCmCmAmAmCfUfAfCmCmGmAmGmUmCmCmGmAm | 259 | VPUmsCfsGmGmAmCfUmCmGmGmUmAfGmUfUmGfGmAmCmsUmsUm |
| TJR101333 | 206 | CmsAmsUmCmAmCmAfGfCfCmCmGmGmAmUmUmGmGmAm | 260 | VPUmsCfsCmAmAmUfCmCmGmGmGmCfUmGfUmGfAmUmGmsUmsUm |
| TJR101334 | 207 | AmsAmsGmGmUmGmGfGfGfAmCmAmUmGmUmGmUmGmAm | 261 | VPUmsCfsAmCmAmCfAmUmGmUmCmCfCmCfAmCfCmUmUmsAmsUm |
| TJR101335 | 208 | AmsGmsGmUmGmGmGfGfAfCmAmUmGmUmGmUmGmUmAm | 262 | VPUmsAfsCmAmCmAfCmAmUmGmUmCfCmCfCmAfCmCmUmsUmsAm |
| TJR101336 | 209 | GmsGmsUmGmGmGmGfAfCfAmUmGmUmGmUmGmUmCmAm | 263 | VPUmsGfsAmCmAmCfAmCmAmUmGmUfCmCfCmCfAmCmCmsUmsUm |
| TJR101337 | 210 | GmsGmsAmAmGmCmUfAfCfCmCmUmGmCmAmGmGmGmAm | 264 | VPUmsCfsCmCmUmGfCmAmGmGmGmUfAmGfCmUfUmCmCmsCmsUm |

(continued)

| Double strand No. | SEQ ID NO: | SS strand (5'-3') | SEQ ID NO: | AS strand (5'-3') |
|---|---|---|---|---|
| TJR101338 | 211 | GmsAmsAmGmCmUmAfCfCfCmUmGmCmAmGmGmGmAmAm | 265 | VPUmsUfsCmCmCmUfGmCmAmGmGmGfUmAfGmCfUmUmCmsCmsCm |
| TJR101339 | 212 | AmsAmsGmCmUmAmCfCfCfUmGmCmAmGmGmGmAmCmAm | 266 | VPUmsGfsUmCmCmCfUmGmCmAmGmGfGmUfAmGfCmUmUmsCmsCm |
| TJR101340 | 213 | AmsCmsCmAmGmGmAfGfAfCmAmCmCmCmUmGmAmGmAm | 267 | VPUmsCfsUmCmAmGfGmGmUmGmUmCfUmCfCmUfGmGmUmsCmsUm |
| TJR101341 | 214 | CmsCmsAmGmGmAmGfAfCfAmCmCmCmUmGmAmGmAmAm | 268 | VPUmsUfsCmUmCmAfGmGmGmUmGmUfCmUfCmCfUmGmGmsUmsCm |

In Tables 1, 2, and 3, the sequences are shown in the direction from the 5' end to the 3' end (left to right); the lowercase letter m means that the upstream nucleotide adjacent to the letter m is a 2'-methoxy-modified nucleotide; the lowercase letter f means that the upstream nucleotide adjacent to the letter f is a 2'-fluoro-modified nucleotide; the lowercase letter s means that the two nucleosides adjacent to the letter s or a nucleoside linked thereto and the targeting ligand **I-14** are linked by a phosphorothioate diester group; unless otherwise specified, two adjacent nucleosides are linked by a phosphodiester group; unless otherwise specified, the 3' position of the first nucleotide of the 3' end of each strand is hydroxy, and the 5' position of the first nucleotide of the 5' end of each strand is hydroxy. The structures of the 2'-methoxy-modified nucleoside, the 2'-fluoro-modified nucleoside, the phosphorothioate diester group, the phosphodiester group, **I-14, and** VPUm are shown in the table below. When the RNAi agents of the present disclosure are present in the form of a salt, for example, in the form of a sodium salt, the structures of the salt forms corresponding to the structures in Table 4 below also fall within the protection scope of the present disclosure:

Table 4

| Structure | Name |
|---|---|
| 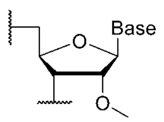 | 2'-Methoxy-modified nucleoside |
| 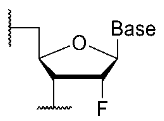 | 2'-Fluoro-modified nucleoside |
| 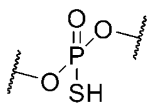 | Phosphorothioate diester group |
| 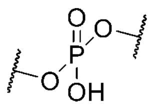 | Phosphodiester group |

(continued)

| Structure | Name |
|---|---|
| 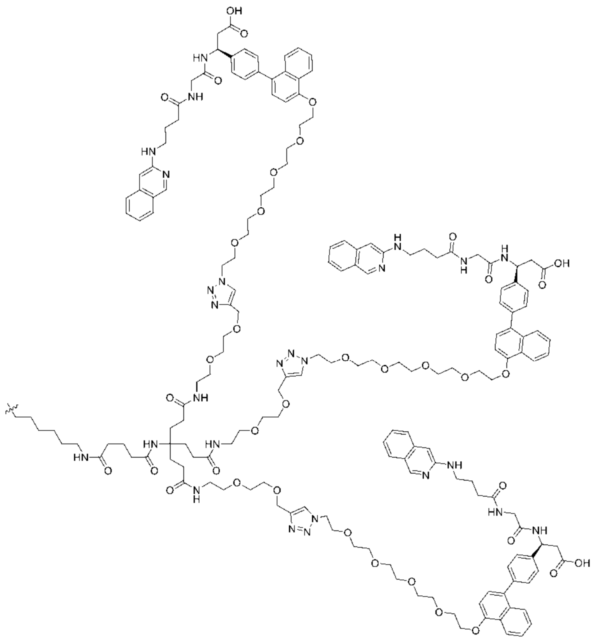 | **I-14** |
| 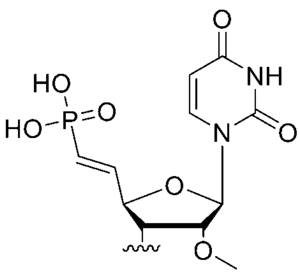 | VPUm |

## Example 2. Synthesis of RNAi Agents

The synthesis of RNAi agents does not differ from the conventional phosphoramidite solid-phase synthesis method. The synthesis process is briefly described below: Nucleoside phosphoramidite monomers were linked one by one according to a synthesis program on a Dr. Oligo48 synthesizer (Biolytic), with a universal CPG support as a start. The nucleoside monomer starting materials 2'-F RNA, 2'-O-methyl RNA, and other nucleoside phosphoramidite monomers were purchased from Shanghai Hongene or Suzhou Genepharma, and the nucleoside phosphoramidite monomers of VPUm were purchased from Jiangsu Synthgene. 5-Ethylthio-1H-tetrazole (ETT) was used as an activator (0.6 M in acetonitrile), a 0.22 M solution of PADS in acetonitrile and collidine (1:1 by volume) (Suzhou Kroma) was used as a sulfurizing agent, and an iodopyridine/water solution (Kroma) was used as an oxidant.

After solid-phase synthesis was complete, the oligoribonucleotides were cleaved from the solid support by soaking in a solution of 28% ammonia water and ethanol (3:1) at 50 °C for 16 h. Centrifugation was then performed, and the supernatant was transferred to another centrifuge tube. After the supernatant was concentrated to dryness by evaporation, the residue was purified by C18 reversed-phase chromatography with 0.1 M TEAA and acetonitrile as the mobile phase, and DMTr was removed using a 3% trifluoroacetic acid solution. The target oligonucleotides were collected, then lyophilized, identified as the target products by LC-MS, and quantified by UV (260 nm).

The single-stranded oligonucleotides obtained were complementarily paired in an equimolar ratio and annealed. The final RNAi agents were dissolved in 1× PBS, and the concentration of the solutions was adjusted to the concentration required for the experiment so that they were ready for use.

## Example 3. Synthesis of Ligand Compound I-12

The synthesis scheme for compound **I-12** is as follows:

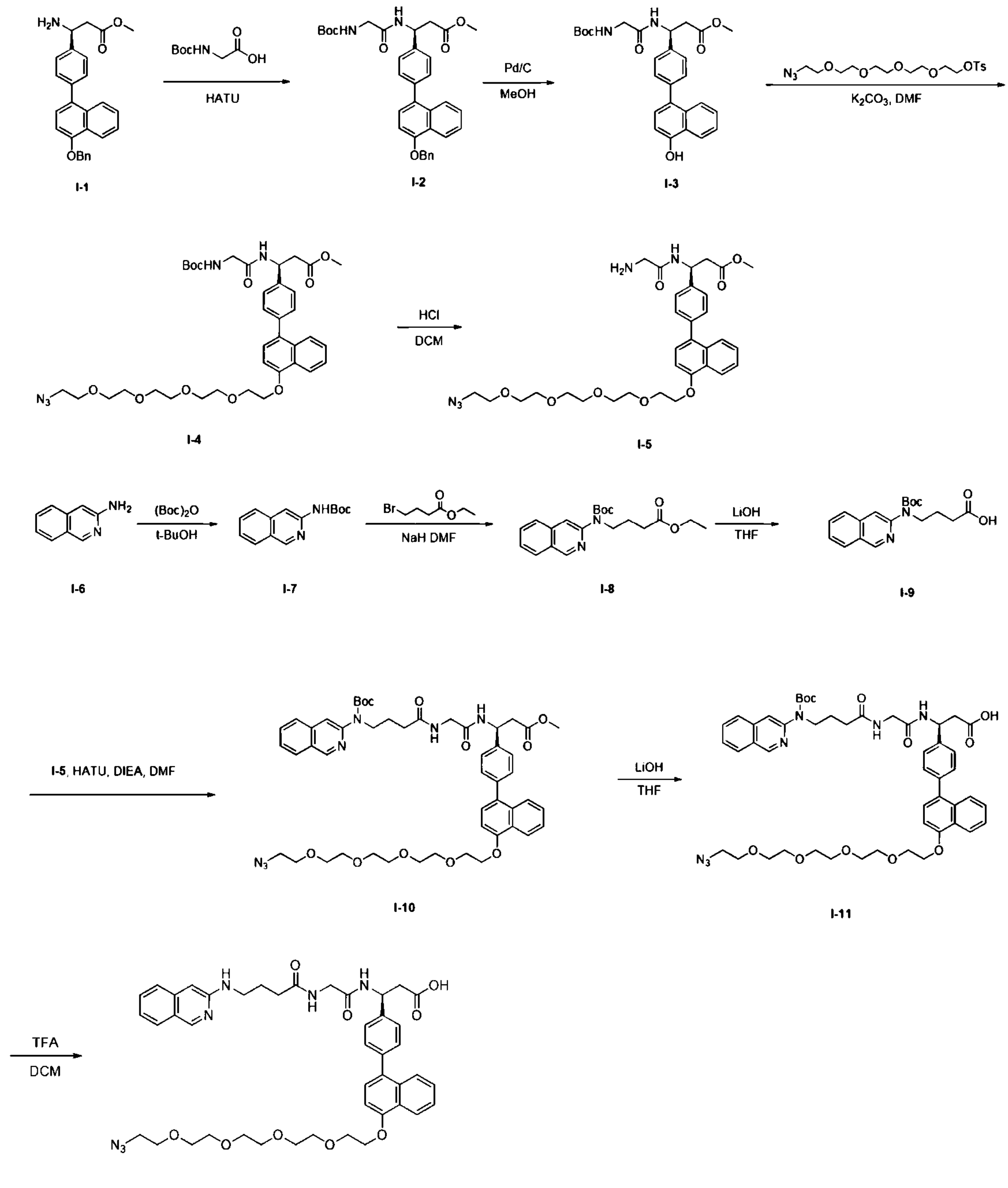


I-1 I-2 I-3 I-4 I-5 I-6 I-7 I-8 I-9 I-10 I-11 I-12

3-1. Synthesis of compound **I-2**

N,N-Diisopropylethylamine (132 mL, 799.2 mmol), O-(7-azabenzotriazol-1-yl)-N,N,N',N'-tetramethyluronium hexafluorophosphate (66.85 g, 175.8 mmol), and compound **I-1** (78.93 g, 191.8 mmol, prepared using the method described in Patent Application No. WO2019089765A1) were added to a solution of N-(tert-butoxycarbonyl)glycine (28.00 g, 159.8 mmol, commercially available) in N-N-dimethylformamide (200 mL), and the mixture was purged 3 times with nitrogen. The reaction was stirred at room temperature for 3 h in a nitrogen atmosphere, and LCMS monitoring showed that the reaction was complete. The reaction mixture was quenched with water and extracted with ethyl acetate. The combined organic phases were washed with saturated brine and dried over anhydrous sodium sulfate. The concentrated crude product was purified by normal-phase silica gel column chromatography to give compound **I-2** (60.0 g, yield: 66%).

LCMS: MS (ESI) m/z = 513.6 $[M+H-56]^+$.

### 3-2. Synthesis of compound I-3

10% palladium on carbon (0.23 g) was added to a solution of compound **I-2** (2.30 g, 4.05 mmol) in methanol (30.0 mL) in a hydrogen atmosphere. The reaction was stirred at room temperature for 8 h, and LCMS monitoring showed that the reaction was complete. The reaction mixture was filtered and concentrated to give compound **I-3** (1.80 g, yield: 93%). $^1$H NMR (400 MHz, DMSO) δ 10.27 (s, 1H), 8.39 (d, *J* = 8.4 Hz, 1H), 8.30-8.09 (m, 1H), 7.75 (dd, *J* = 6.4, 3.2 Hz, 1H), 7.54-7.40 (m, 4H), 7.37 (d, *J* = 8.4 Hz, 2H), 7.23 (d, *J* = 7.6 Hz, 1H), 6.95 (dd, *J* = 14.0, 6.8 Hz, 2H), 5.32 (q, *J* = 7.6 Hz, 1H), 3.60 (s, 3H), 3.57 (d, *J* = 6.0 Hz, 2H), 2.88 (t, *J* = 6.0 Hz, 2H), 1.39 (s, 9H).

### 3-3. Synthesis of compound I-4

Azide-PEG5-Tos (1.74 g, 4.18 mmol) and potassium carbonate (0.58 g, 4.18 mmol) were added to a solution of compound **I-3** (1.00 g, 2.09 mmol) in N,N-dimethylformamide (10.0 mL). The reaction was heated to 80 °C and stirred overnight, and LCMS monitoring showed that the reaction was complete. The reaction mixture was quenched with a saturated sodium bicarbonate solution and extracted with ethyl acetate (3 times, 40 mL each time). The combined organic phases were washed with saturated brine and dried over anhydrous sodium sulfate. The concentrated crude product was purified by normal-phase silica gel column chromatography to give compound **I-4** (1.30, yield: 85%). LCMS: MS (ESI) m/z = 724.5 $[M+H]^+$.

### 3-4. Synthesis of compound I-5

A solution of hydrochloric acid in 1,4-dioxane (20.0 mL) was added to a solution of compound **I-4** (1.30 g, 1.79 mmol) in dichloromethane (20.0 mL). The reaction was stirred at room temperature overnight, and LCMS monitoring showed that the reaction was complete. The reaction mixture was concentrated to give compound **I-5** (1.10 g, yield: 98%). LCMS: MS (ESI) m/z = 624.4 $[M+H]^+$.

### 3-5. Synthesis of compound I-7

Di-tert-butyl dicarbonate (6.58 g, 30.17 mmol) was added to a solution of compound **I-6** (2.90 g, 20.11 mmol, commercially available) in tert-butanol (50.0 mL). The reaction was stirred at room temperature overnight, and LCMS analysis showed that the reaction was complete. The reaction mixture was concentrated to give a crude product, and the crude product was purified by normal-phase silica gel column chromatography to give compound **I-7** (3.90 g, yield: 79%). LCMS: MS (ESI) m/z = 245.4 $[M+H]^+$.

### 3-6. Synthesis of compound I-8

Sodium hydride (0.19 g, 4.80 mmol) was added to a solution of compound **I-7** (0.98 g, 4.00 mmol) in N,N-dimethylformamide (10.0 mL) at 0 °C. After the mixture was stirred for 30 min with the temperature maintained, ethyl 4-bromobutyrate (0.93 mL, 4.81 mmol) was added. The reaction was stirred at room temperature overnight, and LCMS monitoring showed that the reaction was complete. The reaction mixture was quenched with water and extracted with ethyl acetate (3 times, 50 mL each time). The combined organic phases were washed with saturated brine and dried over anhydrous sodium sulfate. The concentrated crude product was purified by normal-phase silica gel column chromatography to give compound **I-8** (1.30 g, yield: 91%). LCMS: MS (ESI) m/z = 359.5 $[M+H]^+$.

### 3-7. Synthesis of compound I-9

A 1 N solution of lithium hydroxide (20.0 mL) was added to a solution of compound **I-8** (1.30 g, 3.63 mmol) in tetrahydrofuran (20.0 mL). The reaction was stirred at room temperature for 2 h, and LCMS monitoring showed that the reaction was complete. The pH of the reaction mixture was adjusted to 2 with 1 N HCl, and extraction was performed with ethyl acetate (3 times, 20 mL each time). The combined organic phases were washed with saturated brine and dried over anhydrous sodium sulfate. The filtrate was concentrated to give compound **I-9** (1.18 g, yield: 98%). LCMS: MS (ESI) m/z = 331.1 $[M+H]^+$.

### 3-8. Synthesis of compound I-10

N,N-Diisopropylethylamine (0.39 g, 3.03 mmol), O-(7-azabenzotriazol-1-yl)-N,N,N',N'-tetramethyluronium hexafluorophosphate (0.28 g, 0.73 mmol), and compound **I-5** (0.28 g, 0.72 mmol) were added to compound **I-9** (0.20 g, 0.61 mmol) in N-N-dimethylformamide (5.0 mL). The reaction was stirred at room temperature for 2 h, and LCMS monitoring

showed that the reaction was complete. The reaction mixture was quenched with water and extracted with ethyl acetate (3 times, 40 mL each time). The combined organic phases were washed with saturated brine and dried over anhydrous sodium sulfate. The concentrated crude product was purified by normal-phase silica gel column chromatography to give compound **I-10**(0.30 g, yield: 53%). LCMS: MS (ESI) m/z = 936.7 $[M+H]^+$.

### 3-9. Synthesis of compound I-11

A 1 N solution of lithium hydroxide (2.0 mL) was added to a solution of compound **I-10** (0.07 g, 0.08 mmol) in tetrahydrofuran (2.0 mL). The reaction was stirred at room temperature for 2 h, and LCMS monitoring showed that the reaction was complete. The pH of the reaction mixture was adjusted to 2 with 1 N HCl, and extraction was performed with ethyl acetate (3 times, 20 mL each time). The combined organic phases were washed with saturated brine and dried over anhydrous sodium sulfate. The filtrate was concentrated to give compound **I-11**(0.27 g, yield: 91%). LCMS: MS (ESI) m/z = 922.1 $[M+H]^+$.

### 3-10. Synthesis of compound I-12

Trifluoroacetic acid (4.0 mL) was added to a solution of compound **I-11**(0.27 g, 0.29 mmol) in dichloromethane (4.0 mL). The reaction was stirred at room temperature for 3 h, and LCMS analysis showed that the reaction was complete. The reaction mixture was concentrated to give a crude product, and the crude product was purified by preparative high performance liquid chromatography to give compound **I-12**(0.09 g, yield: 37%).

LCMS: MS (ESI) m/z = 822.1 $[M+H]^+$.

$^1$H NMR (400 MHz, DMSO) δ 8.97 (s, 1H), 8.51 (d, *J* = 8.4 Hz, 1H), 8.33-8.23 (m, 1H), 8.15 (t, *J* = 5.6 Hz, 1H), 7.88 (d, *J* = 8.4 Hz, 1H), 7.79-7.72 (m, 1H), 7.63 (d, *J* = 8.4 Hz, 1H), 7.51 (m, 8H), 7.31 (d, *J* = 8.0 Hz, 1H), 7.26-7.20 (m, 1H), 7.04 (d, *J* = 8.0 Hz, 1H), 6.91 (s, 1H), 5.32 (q, *J* = 7.2 Hz, 1H), 4.34-4.31 (m, 2H), 3.95-3.91 (m, 2H), 3.78 (d, *J* = 5.6 Hz, 2H), 3.69 (dd, *J* = 6.0, 3.6 Hz, 2H), 3.61-3.50 (m, 13H), 3.38-3.34 (m, 2H), 3.29 (t, *J* = 7.2 Hz, 2H), 2.80 (d, *J*= 7.2 Hz, 2H), 2.29 (t, *J* = 7.2 Hz, 2H), 1.86 (p, *J* = 7.2 Hz, 2H).

The ability of compound **I-12** to bind to the αvβ6 protein was determined by the conventional fluorescence polarization (FP) method in the art, and the $IC_{50}$ value of the binding of compound **I-12** to the human recombinant αvβ6 protein was determined to be 79.86 nM, indicating that compound **I-12** is a ligand capable of binding to the αvβ6 protein. The specific determination method was as follows:
3.5 μL of the test compound **I-12** was added to a 384-well black plate. For the test compound, 12 concentration points were set. The highest concentration was 10 μM, and a 3-fold serial dilution was performed. DMSO control wells were used to determine the maximum signal, and 500 nM compound CWHM-12 (MCE, HY-18644) control wells were used as the minimum signal. Then, 3.5 μL of a 4× human recombinant αvβ6 protein working solution was added. After 15 min of incubation at room temperature, 7 μL of a 2× fluorescent RGD peptide solution was added to each well. The plate was incubated at room temperature for 1 h, and then FP signals were read using Envision. An inhibition curve was fit using GraphPad Prism, and the $IC_{50}$ value was calculated. The $IC_{50}$ value (Z prime > 0.5) was calculated using the following formula:

Curve fitting formula: Y = Bottom + (Top - Bottom) / (1 + 10^((LogIC50 - X) × Hill Slope))

X: Log inhibitor concentration; Y: inhibition rate %.

## Example 4. Synthesis of RNAi Agents with Ligand I-14

### 4-1. Synthesis of sense strands linked to I-13'

The sense strands containing **I-13'** in Table 5 were synthesized using the method in Example 2, with the only difference being that at the position corresponding to **1-13',** the alkynyl-containing phosphoramidite monomer **I-13**, prepared using the method described in Patent Application No. WO2019161213A, was used in the solid-phase synthesis to obtain the sense strands containing **1-13'.** The structures of **I-13** and **I-13'** are shown below:

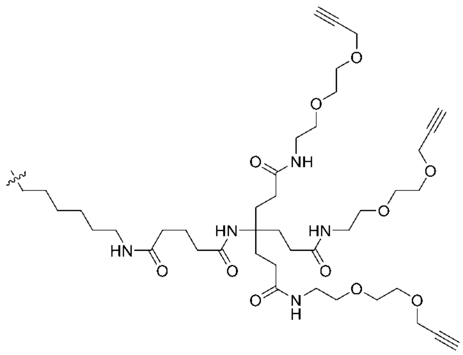

I-13'

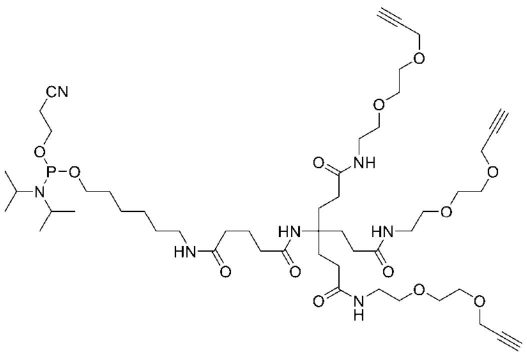

I-13

.

### 4-2. Synthesis of sense strands containing I-14

Stock solutions of 0.5 M tris(3-hydroxypropyltriazolylmethyl)amine (THPTA), 0.5 M Cu(II) sulfate pentahydrate (Cu(II)SO4·5H2O), and 2 M sodium ascorbate solutions were prepared in deionized water. In addition, a 2 M solution of I-13-21 (2.5 μmol), prepared in step **4-1,** in TEAA (10,000 ng/μL) and a 25 mmol/L solution of **I-12** (35 μmol) in DMSO were prepared. The solution of **I-12** in DMSO was added to the 2 M solution of **I-13-21** in TEAA, and the mixture was shaken until it was homogeneous. Then, 75 μL of 0.5 M Cu(II) (Cu(II)$SO_4$ 5$H_2$O), 75 μL of 0.5 M THPTA, and 18 μL of 2 M ascorbate were sequentially added to a centrifuge tube. After 1 h of shaking on a thermostatic reactor, LCMS analysis showed that the reaction was complete. After centrifugation, the supernatant was collected and purified by hydrophobic chromatography (A: ammonium sulfate buffer, B: purified water) to give the sense strand containing **I-14** set forth in SEQ ID NO: 21.

The sense strands set forth in SEQ ID NO: 22-SEQ ID NO: 26 were prepared using the method described above, with the only difference being that the sense strands of **I-13-22** to **I-13-26** were used in place of **I-13-21** in the aforementioned step.

Table 5. The sequences of sense strands linked to I-13' or I-14

| No. | SS strand (5'-3') | SEQ ID NO |
|---|---|---|
| I-13-21 | **I-13'**CmsUmsGmUmCmAmGfCfAfUmCmAmGmCmAmUmCmsAmsAm | 269 |
| I-13-22 | **I-13'**GmsAmsCmCmAmAmCfUfCfUmCmUmCmCmUmGmUmsAmsAm | 270 |
| I-13-23 | **I-13'**AmsGmsAmGmGmAmGfGfAfGmCmGmUmGmCmAmGmsAmsAm | 271 |
| I-13-24 | **I-13'**CmsAmsGmUmCmGmGfAfGfCmUmAmAmUmGmGmUmsGmsAm | 272 |
| I-13-25 | **I-13'**CmsUmsUmUmUmCmUfUfUfUmUmCmCmCmUmUmGmsAmsAm | 273 |
| I-13-26 | **I-13'**CmsCmsUmUmUmUmCfUfUfUmUmUmCmCmCmUmUmsGmsAm | 274 |
| | **I-14**CmsUmsGmUmCmAmGfCfAfUmCmAmGmCmAmUmCmsAmsAm | 21 |
| | **I-14**GmsAmsCmCmAmAmCfUfCfUmCmUmCmCmUmGmUmsAmsAm | 22 |
| | **I-14**AmsGmsAmGmGmAmGfGfAfGmCmGmUmGmCmAmGmsAmsAm | 23 |

(continued)

| No. | SS strand (5'-3') | SEQ ID NO |
|---|---|---|
| | **I-14**CmsAmsGmUmCmGmGfAfGfCmUmAmAmUmGmGmUmsGmsAm | 24 |
| | **I-14**CmsUmsUmUmUmCmUfUfUfUmUmCmCmCmUmUmGmsAmsAm | 25 |
| | **I-14**CmsCmsUmUmUmUmCfUfUfUmUmUmCmCmCmUmUmsGmsAm | 26 |

**4-3. Synthesis of RNAi agents containing I-14**

By using the method in Example 2, the sense strands containing **I-14** obtained in step 4-2 and the corresponding antisense strands were complementarily paired in an equimolar ratio. After annealing, RNAi agents containing **I-14** were obtained.

**Example 5. psiCHECK On-Target Activity of RNAi Agents at 9 Concentration Points**

RNAi agents were subjected to an *in vitro* molecular-level simulation of on-target activity screening in HEK293A cells using 9 concentration gradients.

HEK293A cells were cultured at 37 °C with 5% $CO_2$ in a DMEM high-glucose culture medium containing 10% fetal bovine serum. 24 h prior to transfection, the HEK293A cells were seeded into a 96-well plate at a density of $1 \times 10^4$ cells per well, with each well containing 100 μL of the culture medium.

The cells were co-transfected with the RNAi agents and corresponding plasmids using Lipofectamine2000 (ThermoFisher, 11668019) according to the instructions, with 0.3 μL of Lipofectamine2000 used per well. The amount of plasmids for transfection was 20 ng per well. For on-target sequence plasmids, a total of 9 concentration points were set for the RNAi agents; the highest concentration point final concentration was 20 nM, and a 3-fold serial dilution was performed to obtain 20.0000 nM, 6.6667 nM, 2.2222 nM, 0.7407 nM, 0.2469 nM, 0.0823 nM, 0.0274 nM, 0.0091 nM, and 0.0030 nM. 24 h after transfection, the on-target levels were determined using a Dual-Luciferase Reporter Assay System (Promega, E2940). The results are shown in Table 6.

Table 6. The activity of RNA agents at 9 points in a psiCHECK system

| Remaining percentage of target gene mRNA expression (mean) | | | | | |
|---|---|---|---|---|---|
| Double strand code | 20.0000 nM | 6.6667 nM | 2.2222 nM | 0.7407 nM | 0.2469 nM |
| TJR101283 | 25.27 | 17.14 | 16.25 | 17.00 | 22.23 |
| TJR101284 | 28.51 | 23.36 | 22.84 | 23.30 | 25.43 |
| TJR101285 | NA | 31.47 | 26.57 | 25.96 | 35.92 |
| TJR101287 | NA | NA | 48.98 | 45.72 | 54.97 |
| TJR101288 | 24.84 | 21.93 | 23.06 | 26.27 | 36.07 |
| TJR101289 | 23.07 | 20.74 | 22.68 | 24.42 | 33.10 |
| TJR101290 | 22.35 | 20.74 | 24.73 | 25.82 | 36.92 |
| TJR101291 | 22.74 | 16.76 | 17.99 | 18.60 | 26.95 |
| TJR101296 | 43.55 | 37.53 | 38.41 | 41.47 | 49.01 |
| TJR101299 | 28.47 | 19.94 | 19.56 | 21.97 | 30.07 |
| TJR101302 | NA | 47.83 | 38.55 | 35.14 | 39.75 |
| TJR101303 | 31.16 | 24.10 | 24.41 | 25.27 | 31.38 |
| TJR101304 | 37.65 | 30.28 | 38.05 | 41.70 | 53.19 |
| TJR101306 | 27.81 | 18.58 | 16.24 | 17.68 | 23.76 |

(continued)

| Remaining percentage of target gene mRNA expression (mean) | | | | | |
|---|---|---|---|---|---|
| Double strand code | 20.0000 nM | 6.6667 nM | 2.2222 nM | 0.7407 nM | 0.2469 nM |
| TJR101307 | 43.07 | 34.76 | 38.00 | 37.43 | 46.41 |
| TJR101308 | 34.04 | 22.41 | 21.78 | 24.09 | 28.63 |
| TJR101309 | NA | 45.26 | 36.07 | 32.79 | 41.19 |
| TJR101313 | NA | 41.99 | 38.67 | 39.22 | 51.43 |
| TJR101314 | 39.31 | 23.76 | 22.74 | 23.56 | 32.63 |
| TJR101316 | NA | 43.82 | 41.74 | 41.39 | 51.77 |
| TJR101318 | 33.75 | 21.60 | 18.71 | 17.10 | 25.42 |
| TJR101319 | NA | 14.01 | 10.67 | 11.11 | 18.01 |
| TJR101320 | 30.76 | 21.38 | 20.87 | 21.39 | 30.62 |
| TJR101321 | 29.44 | 22.08 | 23.26 | 25.49 | 35.15 |
| TJR101322 | 19.27 | 15.75 | 15.06 | 16.33 | 19.49 |
| TJR101325 | 50.76 | 50.57 | 46.11 | 46.87 | 53.12 |
| TJR101327 | 47.89 | 43.61 | 49.01 | 52.48 | 61.16 |
| TJR101329 | NA | 47.09 | 42.14 | 44.17 | 63.15 |
| TJR101331 | 39.80 | 30.02 | 25.63 | 26.87 | 38.09 |
| TJR101332 | NA | 43.79 | 48.83 | 52.19 | 61.91 |
| TJR101334 | 48.12 | 47.65 | 45.49 | 45.47 | 51.47 |
| TJR101338 | 37.68 | 32.68 | 35.62 | 41.00 | 61.64 |
| TJR101340 | 32.03 | 33.66 | 34.16 | 38.88 | 53.00 |
| TJR101341 | 58.65 | 49.46 | 46.82 | 58.39 | 73.74 |
| Double strand code | 0.0823 nM | 0.0274 nM | 0.0091 nM | 0.0030 nM | $IC_{50}$ (nM) |
| TJR101283 | 30.32 | 54.37 | 81.97 | 96.32 | 0.03 |
| TJR101284 | 32.95 | 53.37 | 79.54 | 91.13 | 0.03 |
| TJR101285 | 63.65 | 83.50 | 96.16 | 103.70 | 0.13 |
| TJR101287 | 70.76 | 87.91 | 87.15 | 92.31 | 0.42 |
| TJR101288 | 56.81 | 80.08 | 90.31 | 99.19 | 0.11 |
| TJR101289 | 55.33 | 86.49 | 89.47 | 100.14 | 0.11 |
| TJR101290 | 60.47 | 86.33 | 96.29 | 95.99 | 0.13 |
| TJR101291 | 43.45 | 70.39 | 87.80 | 93.99 | 0.06 |
| TJR101296 | 68.56 | 83.72 | 94.66 | 92.75 | 0.23 |
| TJR101299 | 49.44 | 74.23 | 92.66 | 98.68 | 0.08 |
| TJR101302 | 56.69 | 74.24 | 91.14 | 106.34 | 0.11 |
| TJR101303 | 44.97 | 68.91 | 89.00 | 112.99 | 0.06 |
| TJR101304 | 70.37 | 92.27 | 96.31 | 111.37 | 0.32 |
| TJR101306 | 33.84 | 53.18 | 76.48 | 93.25 | 0.03 |
| TJR101307 | 56.69 | 73.02 | 88.77 | 97.49 | 0.15 |
| TJR101308 | 39.63 | 61.63 | 83.21 | 92.65 | 0.05 |
| TJR101309 | 58.05 | 82.17 | 88.88 | 93.73 | 0.12 |

(continued)

| Double strand code | 0.0823 nM | 0.0274 nM | 0.0091 nM | 0.0030 nM | $IC_{50}$ (nM) |
|---|---|---|---|---|---|
| TJR101313 | 63.26 | 86.83 | 93.23 | 98.69 | 0.22 |
| TJR101314 | 51.07 | 79.22 | 83.03 | 90.30 | 0.09 |
| TJR101316 | 71.42 | 93.56 | 100.63 | 108.46 | 0.28 |
| TJR101318 | 43.06 | 75.70 | 87.46 | 100.71 | 0.06 |
| TJR101319 | 34.69 | 63.36 | 86.12 | 88.40 | 0.05 |
| TJR101320 | 52.48 | 82.77 | 100.04 | 105.66 | 0.09 |
| TJR101321 | 52.40 | 80.15 | 100.37 | 106.47 | 0.09 |
| TJR101322 | 26.41 | 51.24 | 74.65 | 82.50 | 0.03 |
| TJR101325 | 65.05 | 83.67 | 94.06 | 91.80 | 0.44 |
| TJR101327 | 78.20 | 96.90 | 102.19 | 105.07 | 0.99 |
| TJR101329 | 80.88 | 88.09 | 102.51 | 100.68 | 0.92 |
| TJR101331 | 56.51 | 86.07 | 98.27 | 108.03 | 0.11 |
| TJR101332 | 82.66 | 96.34 | 96.12 | 101.44 | 0.87 |
| TJR101334 | 73.65 | 85.46 | 90.21 | 91.01 | 0.31 |
| TJR101338 | 81.92 | 94.68 | 98.55 | 103.23 | 0.42 |
| TJR101340 | 81.08 | 95.38 | 108.91 | 105.06 | 0.30 |
| TJR101341 | 97.54 | 100.83 | 111.51 | 109.09 | 2.02 |

In Table 6, NA means that no detection was performed.

**Example 6. psiCHECK On-Target Activity of RNAi Agents at 9 Concentration Points**

RNAi agents were subjected to an *in vitro* molecular-level simulation of on-target activity screening in HEK293A cells using 9 concentration gradients.

HEK293A cells were cultured at 37 °C with 5% $CO_2$ in a DMEM high-glucose culture medium containing 10% fetal bovine serum. 24 h prior to transfection, the HEK293A cells were seeded into a 96-well plate at a density of $1 \times 10^4$ cells per well, with each well containing 100 μL of the culture medium.

The cells were co-transfected with the RNAi agents and corresponding plasmids using Lipofectamine2000 (ThermoFisher, 11668019) according to the instructions, with 0.3 μL of Lipofectamine2000 used per well. The amount of plasmids for transfection was 20 ng per well. For on-target sequence plasmids, a total of 9 concentration points were set for the RNAi agents; the highest concentration point final concentration was 20 nM, and a 3-fold serial dilution was performed to obtain 20.0000 nM, 6.6667 nM, 2.2222 nM, 0.7407 nM, 0.2469 nM, 0.0823 nM, 0.0274 nM, 0.0091 nM, and 0.0030 nM. 24 h after transfection, the on-target levels were determined using a Dual-Luciferase Reporter Assay System (Promega, E2940). The results are shown in Table 7.

Table 7. The activity of RNA agents at 9 points in a psiCHECK system

| Remaining percentage of target gene mRNA expression (mean) | | | | | |
|---|---|---|---|---|---|
| Double strand code | 20.0000 nM | 6.6667 nM | 2.2222 nM | 0.7407 nM | 0.2469 nM |
| TJR101282 | 33.21 | 24.50 | 28.41 | 35.45 | 52.04 |
| TJR101293 | 15.86 | 15.93 | 20.53 | 27.57 | 43.93 |
| TJR101298 | 24.83 | 23.17 | 26.64 | 33.26 | 51.19 |
| TJR101301 | 20.76 | 19.85 | 21.79 | 24.54 | 37.75 |
| TJR101305 | 22.43 | 22.93 | 28.95 | 32.62 | 40.29 |
| TJR101311 | 43.00 | 46.55 | 52.53 | 57.50 | 73.52 |

(continued)

| Remaining percentage of target gene mRNA expression (mean) | | | | | |
|---|---|---|---|---|---|
| Double strand code | 20.0000 nM | 6.6667 nM | 2.2222 nM | 0.7407 nM | 0.2469 nM |
| TJR101315 | 45.03 | 44.29 | 57.12 | 65.85 | 78.76 |
| TJR101317 | 35.44 | 36.63 | 39.55 | 42.79 | 52.20 |
| Double strand code | 0.0823 nM | 0.0274 nM | 0.0091 nM | 0.0030 nM | $IC_{50}$ (nM) |
| TJR101282 | 78.04 | 93.22 | 99.60 | 102.40 | 0.27 |
| TJR101293 | 69.87 | 87.87 | 95.25 | 98.32 | 0.19 |
| TJR101298 | 75.47 | 93.28 | 99.29 | 104.83 | 0.26 |
| TJR101301 | 64.20 | 85.60 | 95.52 | 97.40 | 0.14 |
| TJR101305 | 58.17 | 81.50 | 95.03 | 99.56 | 0.14 |
| TJR101311 | 91.41 | 89.15 | 92.50 | 92.73 | 1.86 |
| TJR101315 | 90.93 | 97.56 | 101.67 | 102.73 | 4.23 |
| TJR101317 | 73.44 | 85.12 | 94.44 | 99.24 | 0.34 |

**Example 7. Endogenous Cell Activity Screening of RNAi Agents**

For some sequences, RNAi agents were subjected to a molecular-level simulation of endogenous cell activity screening in BEAS-2B cells using 3 concentration gradients. For the other sequences, RNAi agents were subjected to a molecular-level simulation of endogenous cell activity screening in BEAS-2B cells using 7 concentration gradients. BEAS-2B cells were cultured in a BEAS-2B complete culture medium (Nanjing Cobioer, CC-3170) at 37 °C with 5% $CO_2$. 24 h prior to transfection, the BEAS-2B cells were seeded into a 96-well plate at a density of $1 \times 10^4$ cells per well, with each well containing 100 $\mu$L of the culture medium.

The cells were transfected with the RNAi agents using an RNAi MAX transfection reagent (ThermoFisher, 13778150) according to the instructions, with 0.3 $\mu$L of the RNAi MAX transfection reagent used per well. For some RNAi agents, 3 concentration points were set; the highest concentration point final concentration was 20 nM, and a 5-fold serial dilution was performed to obtain 20 nM, 4 nM, and 0.8 nM. For the other RNAi agents, a total of 7 concentration points were set; the highest concentration point final concentration was 100 nM, and a 5-fold serial dilution was performed to obtain 100.0000 nM, 20 nM, 4 nM, 0.8 nM, 0.16 nM, 0.032 nM, and 0.0064 nM. 48 h after transfection, total cell RNA extraction was performed using a high-throughput cell RNA extraction kit (ThermoFisher, A27828), and RNA reverse transcription (Takara, RR037B) and quantitative real-time PCR detection (ThermoFisher, 4444557) were performed to determine the mRNA level of human RAGE. The mRNA level of human RAGE was corrected based on the GAPDH internal reference gene level.

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as $2^{-\triangle\triangle Ct}$, where $\triangle\triangle$Ct = [(target gene of Ct experimental group - internal reference of Ct experimental group) - (target gene of Ct control group - internal reference of Ct control group)]. Inhibition rate (%) = (1 - remaining level of target gene expression) $\times$ 100%. The results are expressed relative to the remaining percentage of human RAGE expression in cells treated with the dsRNAs. The inhibition rate $IC_{50}$ results are shown in Table 8:

Table 8. The $IC_{50}$ results of RNAi agents in BEAS-2B cells

| Compound No. | Remaining percentage of target gene mRNA expression (mean) | | | | | | | $IC_{50}$ value (nM) |
|---|---|---|---|---|---|---|---|---|
| | 100 nM | 20 nM | 4 nM | 0.8 nM | 0.16 nM | 0.032 nM | 0.0064 nM | |
| TJR101303 | 28.47 | 36.14 | 33.29 | 42.00 | 82.05 | 102.31 | 106.55 | 0.52 |
| TJR101314 | NA | 31.61 | 24.51 | 35.47 | 70.87 | 98.44 | 101.85 | 0.33 |
| TJR101317 | 46.62 | 41.97 | 37.83 | 62.29 | 89.89 | 95.27 | 87.96 | 0.94 |

In Table 8, NA means that no detection was performed.

**Example 8. psiCHECK On-Target Activity of RNAi Agents at 9 Concentration Points**

RNAi agents were subjected to an *in vitro* molecular-level simulation of on-target activity screening in HEK293A cells using 9 concentration gradients.

HEK293A cells were cultured at 37 °C with 5% $CO_2$ in a DMEM high-glucose culture medium containing 10% fetal bovine serum. 24 h prior to transfection, the HEK293A cells were seeded into a 96-well plate at a density of $1 \times 10^4$ cells per well, with each well containing 100 μL of the culture medium.

The cells were co-transfected with the RNAi agents and corresponding plasmids using Lipofectamine2000 (ThermoFisher, 11668019) according to the instructions, with 0.3 μL of Lipofectamine2000 used per well. The amount of plasmids for transfection was 20 ng per well. For on-target sequence plasmids, a total of 9 concentration points were set for the RNAi agents; the highest concentration point final concentration was 20 nM, and a 3-fold serial dilution was performed to obtain 20.0000 nM, 6.6667 nM, 2.2222 nM, 0.7407 nM, 0.2469 nM, 0.0823 nM, 0.0274 nM, 0.0091 nM, and 0.0030 nM. 24 h after transfection, the on-target levels were determined using a Dual-Luciferase Reporter Assay System (Promega, E2940). The results are shown in Table 9.

Table 9. The IC50 activity of RNAi agents in a psiCHECK system

| Double strand code | $IC_{50}$ (nM) |
|---|---|
| TJR103569 | 0.016 |
| TJR103570 | 0.021 |
| TJR103571 | 0.017 |
| TJR103572 | 0.008 |
| TJR103573 | 0.025 |
| TJR103574 | 0.017 |
| TJR103578 | 0.018 |
| TJR103781 | 0.027 |
| TJR103633-TJ-16 | 0.343 |
| TJR103634-TJ-16 | 0.378 |
| TJR103635-TJ-16 | 0.636 |
| TJR103636-TJ-16 | 0.287 |
| TJR103637-TJ-16 | 0.830 |
| TJR103638-TJ-16 | 0.973 |

**Example 9. Endogenous Cell Activity Screening of RNAi Agents**

For the sequences, RNAi agents were subjected to a molecular-level simulation of endogenous cell activity screening in BEAS-2B cells using 7 concentration gradients.

BEAS-2B cells were cultured in a BEAS-2B complete culture medium (Nanjing Cobioer, CC-3170) at 37 °C with 5% $CO_2$. The cells were transfected with the RNAi agents using an RNAi MAX transfection reagent (ThermoFisher, 13778150) according to the instructions, with 0.3 μL of the RNAi MAX transfection reagent used per well. For the RNAi agents, a total of 7 concentration points were set; the highest concentration point final concentration was 100 nM, and a 5-fold serial dilution was performed to obtain 100.0000 nM, 20 nM, 4 nM, 0.8 nM, 0.16 nM, 0.032 nM, and 0.0064 nM. 48 h after transfection, total cell RNA extraction was performed using a high-throughput cell RNA extraction kit (ThermoFisher, A27828), and RNA reverse transcription (Takara, RR037B) and quantitative real-time PCR detection (ThermoFisher, 4444557) were performed to determine the mRNA level of human RAGE. The mRNA level of human RAGE was corrected based on the GAPDH internal reference gene level.

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as $2^{-\triangle\triangle Ct}$, where $\triangle\triangle Ct$ = [(target gene of Ct

experimental group - internal reference of Ct experimental group) - (target gene of Ct control group - internal reference of Ct control group)]. Inhibition rate (%) = (1 - remaining level of target gene expression) × 100%. The results are expressed relative to the remaining percentage of human RAGE expression in cells treated with the dsRNAs. The inhibition rate $IC_{50}$ results are shown in Table 10:

Table 10. The $IC_{50}$ results of RNAi agents in BEAS-2B cells

| Double strand code | $IC_{50}$ (nM) |
|---|---|
| TJR103569 | 1.311 |
| TJR103575 | >100 |
| TJR103577 | >100 |
| TJR103578 | 2.241 |

## Claims

**1.** An RNAi agent, comprising a sense strand and an antisense strand that form a double-stranded region, wherein:

the sense strand comprises at least 15 contiguous nucleotides and differs from any one of the nucleotide sequences of SEQ ID NO: 1, SEQ ID NO: 7, SEQ ID NO: 2, SEQ ID NO: 8, SEQ ID NO: 3, SEQ ID NO: 4, SEQ ID NO: 5, and SEQ ID NO: 6 by no more than 3 nucleotides; and
the antisense strand comprises at least 15 contiguous nucleotides and differs from any one of the nucleotide sequences of SEQ ID NO: 27, SEQ ID NO: 33, SEQ ID NO: 28, SEQ ID NO: 34, SEQ ID NO: 29, SEQ ID NO: 30, SEQ ID NO: 31, and SEQ ID NO: 32 by no more than 3 nucleotides.

**2.** The RNAi agent according to claim 1, wherein:

the sense strand comprises at least 17 contiguous nucleotides of a sequence differing from any one of the nucleotide sequences of SEQ ID NO: 1 to SEQ ID NO: 8 by no more than 3 nucleotides; and
the antisense strand comprises at least 17 contiguous nucleotides of a sequence differing from any one of the nucleotide sequences of SEQ ID NO: 27 to SEQ ID NO: 34 by no more than 3 nucleotides;
preferably, the sense strand comprises at least 19 contiguous nucleotides of a sequence differing from any one of the nucleotide sequences of SEQ ID NO: 1 to SEQ ID NO: 8 by no more than 3 nucleotides, preferably by no more than one nucleotide, and
the antisense strand comprises at least 21 contiguous nucleotides of a sequence differing from any one of the nucleotide sequences of SEQ ID NO: 27 to SEQ ID NO: 34 by no more than 3 nucleotides, preferably by no more than one nucleotide.

**3.** The RNAi agent according to any one of claims 1-2, comprising a sense strand comprising at least 18 contiguous nucleotides shown in any one of the following groups and an antisense strand comprising at least 20 contiguous nucleotides shown in any one of the following groups:

group 1): a sense strand set forth in SEQ ID NO: 1 and an antisense strand set forth in SEQ ID NO: 27;
group 2): a sense strand set forth in SEQ ID NO: 7 and an antisense strand set forth in SEQ ID NO: 33;
group 3): a sense strand set forth in SEQ ID NO: 2 and an antisense strand set forth in SEQ ID NO: 28;
group 4): a sense strand set forth in SEQ ID NO: 8 and an antisense strand set forth in SEQ ID NO: 34;
group 5): a sense strand set forth in SEQ ID NO: 3 and an antisense strand set forth in SEQ ID NO: 29;
group 6): a sense strand set forth in SEQ ID NO: 4 and an antisense strand set forth in SEQ ID NO: 30;
group 7): a sense strand set forth in SEQ ID NO: 5 and an antisense strand set forth in SEQ ID NO: 31; and
group 8): a sense strand set forth in SEQ ID NO: 6 and an antisense strand set forth in SEQ ID NO: 32;
wherein preferably, the RNAi agent comprises the sense strand and the antisense strand shown in any one of the groups.

**4.** The RNAi agent according to any one of claims 1-3, wherein at least one nucleotide in the sense strand and/or the antisense strand is a modified nucleotide.

5. The RNAi agent according to any one of claims 1-4, wherein:

three contiguous nucleotides in the sense strand are 2'-fluoro-modified nucleotides;
preferably, in the direction from the 5' end to the 3' end, the nucleotides at positions 7, 8, and 9 of the sense strand are 2'-fluoro-modified nucleotides;
and/or
in the direction from the 5' end to the 3' end, each of the nucleotides at positions 2, 6, 12, 14, and 16 of the antisense strand is independently a 2'-fluoro-modified nucleotide, or the nucleotide at position 2, 4, 6, 10, 12, 14, 16, or 18 of the antisense strand is independently a 2'-fluoro-modified nucleotide;
the nucleotides at the remaining positions of the sense strand and the antisense strand are 2'-methoxy-modified nucleotides.

6. The RNAi agent according to any one of claims 1-5, wherein at least one phosphodiester group in the sense strand and/or the antisense strand is a phosphodiester group with a modification group, preferably a phosphorothioate diester group.

7. The RNAi agent according to claim 6, wherein the phosphorothioate diester group is present in at least one of the following positions:

between the 1st and 2nd nucleotides of the 5' end of the sense strand;
between the 2nd and 3rd nucleotides of the 5' end of the sense strand;
between the 1st and 2nd nucleotides of the 3' end of the sense strand;
between the 2nd and 3rd nucleotides of the 3' end of the sense strand;
between the 1st and 2nd nucleotides of the 5' end of the antisense strand;
between the 2nd and 3rd nucleotides of the 5' end of the antisense strand;
between the 1st and 2nd nucleotides of the 3' end of the antisense strand; and
between the 2nd and 3rd nucleotides of the 3' end of the antisense strand;
preferably, the sense strand and/or the antisense strand comprise(s) a plurality of phosphorothioate diester groups, and the phosphorothioate diester groups are present:

between the 1st and 2nd nucleotides of the 5' end of the sense strand; and
between the 2nd and 3rd nucleotides of the 5' end of the sense strand; and
between the 1st and 2nd nucleotides of the 5' end of the antisense strand; and
between the 2nd and 3rd nucleotides of the 5' end of the antisense strand; and
between the 1st and 2nd nucleotides of the 3' end of the antisense strand; and
between the 2nd and 3rd nucleotides of the 3' end of the antisense strand;
or,
the phosphorothioate diester groups are present:

between the 1st and 2nd nucleotides of the 5' end of the sense strand; and
between the 2nd and 3rd nucleotides of the 5' end of the sense strand; and
between the 1st and 2nd nucleotides of the 3' end of the sense strand; and
between the 2nd and 3rd nucleotides of the 3' end of the sense strand; and
between the 1st and 2nd nucleotides of the 5' end of the antisense strand; and
between the 2nd and 3rd nucleotides of the 5' end of the antisense strand; and
between the 1st and 2nd nucleotides of the 3' end of the antisense strand; and
between the 2nd and 3rd nucleotides of the 3' end of the antisense strand.

8. The RNAi agent according to any one of claims 1-7, wherein:
the 5' end of the antisense strand comprises one phosphorus-containing group, and the phosphorus-containing group is a 5'-vinylphosphodiester group.

9. The RNAi agent according to any one of claims 1-8, wherein:

the sense strand comprises the nucleotide sequence set forth in any one of SEQ ID NO: 9 to SEQ ID NO: 20; and,
the antisense strand comprises the nucleotide sequence set forth in any one of SEQ ID NO: 35 to SEQ ID NO: 46.

10. The RNAi agent according to any one of claims 1-9, wherein:

an end of the sense strand is linked to a targeting ligand;
preferably, the 5' end of the sense strand is linked to the targeting ligand.

11. The RNAi agent according to claim 10, wherein:

the targeting ligand has affinity for a cellular receptor expressed on an epithelial cell;
preferably, the targeting ligand is an integrin-targeting ligand;
more preferably, the integrin-targeting ligand is an $\alpha v\beta 6$ integrin-targeting ligand.

12. The RNAi agent according to claim 10 or 11, wherein:

the sense strand comprises the nucleotide sequence set forth in any one of SEQ ID NO: 21 to SEQ ID NO: 26; and
the antisense strand comprises the nucleotide sequence set forth in any one of SEQ ID NO: 47 to SEQ ID NO: 52.

13. A pharmaceutical composition, comprising the RNAi agent according to any one of claims 1-12 and a pharmaceutically acceptable excipient.

14. A cell, comprising the RNAi agent according to any one of claims 1-12.

15. A kit, comprising the RNAi agent according to any one of claims 1-12 or the pharmaceutical composition according to claim 13.

16. A method for reducing the expression of the receptor for advanced glycosylation end-products (RAGE), comprising administering to a subject an effective amount or effective dose of the RNAi agent according to any one of claims 1-12 or the pharmaceutical composition according to claim 13.

17. A method for treating and/or preventing a disease associated with RAGE gene expression in a subject, comprising administering to the subject an effective amount or effective dose of the RNAi agent according to any one of claims 1-12 or the pharmaceutical composition according to claim 13.

18. A method for treating and/or preventing a pulmonary disease, comprising administering to a subject an effective amount or effective dose of the RNAi agent according to any one of claims 1-12 or the pharmaceutical composition according to claim 13,
wherein preferably, the pulmonary disease is selected from the group consisting of asthma, acute respiratory distress syndrome, idiopathic pulmonary fibrosis, chronic obstructive pulmonary disease, cystic fibrosis, and pneumonia.

19. A method for delivering an RNAi agent inhibiting RAGE expression and/or replication to the lungs *in vivo,* comprising administering to a subject the RNAi agent according to any one of claims 1-12 or the pharmaceutical composition according to claim 13.

20. A method for preparing an RNAi agent, comprising: synthesizing the RNAi agent according to any one of claims 1-12.

**INTERNATIONAL SEARCH REPORT**

International application No.
**PCT/CN2024/114147**

**A. CLASSIFICATION OF SUBJECT MATTER**

A61K31/713(2006.01)i; C12N15/113(2010.01)i; A61P11/00(2006.01)i

According to International Patent Classification (IPC) or to both national classification and IPC

**B. FIELDS SEARCHED**

Minimum documentation searched (classification system followed by classification symbols)

IPC: A61K, C12N, A61P

Documentation searched other than minimum documentation to the extent that such documents are included in the fields searched

Electronic data base consulted during the international search (name of data base and, where practicable, search terms used)

CNTXT, WPABSC, WPABS, ENTXTC, ENTXT, DPWI, VEN, CNKI, 万方数据, Wanfang Data, Web of Science, 百度学术, Baidu Scholar, HimmPat, incoPat, 读秀, DUXIU, 中国生物序列检索系统, China Biological Sequence Search System, NCBI, EBI, STNext: 申请人, 发明人, Applicants, Inventors, 晚期糖基化终产物受体, RAGE, AGER , advanced glycosylation end-product specific receptor, dsRNA, siRNA, RNAi, miRNA, 修饰, 靶向, N-乙酰基-半乳糖胺, GalNAc, NAG, modif+, target+, N-acetyl-galactosamine, 序列1, 5, 27, 31, sequences 1, 5, 27 and 31

**C. DOCUMENTS CONSIDERED TO BE RELEVANT**

| Category* | Citation of document, with indication, where appropriate, of the relevant passages | Relevant to claim No. |
|---|---|---|
| A | WO 2022216920 A1 (ARROWHEAD PHARMACEUTICALS, INC.) 13 October 2022 (2022-10-13)<br>description, paragraphs 9, 11-13, 20, 25, 27, 29, 40 and 178, and tables 3 and 11 | 1-20 (in part) |
| A | CN 108251455 A (THE SECOND AFFILIATED HOSPITAL & YUYING CHILDREN'S HOSPITAL OF WENZHOU MEDICAL UNIVERSITY et al.) 06 July 2018 (2018-07-06)<br>description, paragraphs 8, 13, 25, 27, 76 and 96 | 1-20 (in part) |
| A | RADIA, A.M. et al. "Specific siRNA Targeting Receptor for Advanced Glycation End Products (RAGE) Decreases Proliferation in Human Breast Cancer Cell Lines" *International Journal of Molecular Sciences*, Vol. vol. 14, 11 April 2013 (2013-04-11), 7959-7978<br>abstract, and sections 2.2, 2.3 and 4.2 | 1-20 (in part) |
| A | CN 104491877 A (AFFILIATED HOSPITAL OF NANTONG UNIVERSITY) 08 April 2015 (2015-04-08)<br>description, paragraphs 6-9, 14 and 25 | 1-20 (in part) |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 November 2024** | **07 November 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No. **PCT/CN2024/114147**

**C. DOCUMENTS CONSIDERED TO BE RELEVANT**

| Category* | Citation of document, with indication, where appropriate, of the relevant passages | Relevant to claim No. |
|---|---|---|
| A | WO 2013153092 A1 (DEUTSCHES KREBSFORSCHUNGSZENTRUM et al.) 17 October 2013 (2013-10-17)<br>claims 1 and 6, and description, page 3, lines 5-28, and page 8, lines 9-43 | 1-20 (in part) |
| A | HONG, J. et al. "Cardiac RNAi Therapy Using RAGE siRNA/Deoxycholic Acid-modified Polyethylenimine Complexes for Myocardial Infarction"<br>*Biomaterials*, 31 December 2014 (2014-12-31), 1-12<br>abstract, and sections 2.1, 2.2, 3.1 and 3.2 | 1-20 (in part) |

Form PCT/ISA/210 (second sheet) (July 2022)

**INTERNATIONAL SEARCH REPORT**

International application No.
**PCT/CN2024/114147**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No. **PCT/CN2024/114147**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17, 18**
because they relate to subject matter not required to be searched by this Authority, namely:

Claim 17 relates to a method for treating and/or preventing diseases associated with RAGE gene expression in a subject, and claim 18 relates to a method for treating and/or preventing lung diseases, both of which belong to a method for treatment of a living human or animal body, and falls within technical solutions for which no search is required by the International Searching Authority as defined in PCT Rule 39.1(iv). However, the present search is made on the basis of the use of the RNAi agent or pharmaceutical composition in the preparation of a related product.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

Invention I: claims 1-20 (in part) relate to an RNAi agent having a sense strand with a nucleotide sequence as shown in SEQ ID NO: 1 and an antisense strand with a nucleotide sequence as shown in SEQ ID NO: 27, and a pharmaceutical composition, cell, kit and method related thereto.

Invention II: claims 1-20 (in part) relate to an RNAi agent having a sense strand with a nucleotide sequence as shown in SEQ ID NO: 2 and an antisense strand with a nucleotide sequence as shown in SEQ ID NO: 28, and a pharmaceutical composition, cell, kit and method related thereto.

Invention III: claims 1-20 (in part) relate to an RNAi agent having a sense strand with a nucleotide sequence as shown in SEQ ID NO: 3 and an antisense strand with a nucleotide sequence as shown in SEQ ID NO: 29, and a pharmaceutical composition, cell, kit and method related thereto.

Invention IV: claims 1-20 (in part) relate to an RNAi agent having a sense strand with a nucleotide sequence as shown in SEQ ID NO: 4 and an antisense strand with a nucleotide sequence as shown in SEQ ID NO: 30, and a pharmaceutical composition, cell, kit and method related thereto.

Invention V: claims 1-20 (in part) relate to an RNAi agent having a sense strand with a nucleotide sequence as shown in SEQ ID NO: 5 and an antisense strand with a nucleotide sequence as shown in SEQ ID NO: 31, and a pharmaceutical composition, cell, kit and method related thereto.

Invention VI: claims 1-20 (in part) relate to an RNAi agent having a sense strand with a nucleotide sequence as shown in SEQ ID NO: 6 and an antisense strand with a nucleotide sequence as shown in SEQ ID NO: 32, and a pharmaceutical composition, cell, kit and method related thereto.

Invention VII: claims 1-20 (in part) relate to an RNAi agent having a sense strand with a nucleotide sequence as shown in SEQ ID NO: 7 and an antisense strand with a nucleotide sequence as shown in SEQ ID NO: 33, and a pharmaceutical composition, cell, kit and method related thereto.

Invention VIII: claims 1-20 (in part) relate to an RNAi agent having a sense strand with a nucleotide sequence as shown in SEQ ID NO: 8 and an antisense strand with a nucleotide sequence as shown in SEQ ID NO: 34, and a pharmaceutical composition, cell, kit and method related thereto.

The common technical features comprised in inventions I to VIII are: an RNAi capable of inhibiting an RAGE gene, the RNAi containing a sense strand and an antisense strand, which form a double-stranded region. However, the above technical features are disclosed in the prior art in the art, for example, WO 2022216920 A1 (publication date: 13 October 2022) discloses an RNAi agent for inhibiting the expression of a receptor for advanced glycation

INTERNATIONAL SEARCH REPORT

International application No.
**PCT/CN2024/114147**

**Box No. III Observations where unity of invention is lacking (Continuation of item 3 of first sheet)**

endproducts, and a composition thereof and a use method therefor, wherein the RNAi agent comprises a sense strand and an antisense strand (which can form a double-stranded region), and can inhibit the expression of an RAGE gene involving a human subject. Therefore, inventions I to VIII described above do not have a same or corresponding special technical feature, and thus do not comply with PCT Rule 13.2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☑ As only some of the required additional search fees were timely paid by the applicant, this international search report covers only those claims for which fees were paid, specifically claims Nos.: **the technical solutions of claims 1-20 (in part) relating to invention I and invention V**

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

**INTERNATIONAL SEARCH REPORT**
**Information on patent family members**

International application No.
**PCT/CN2024/114147**

| Patent document cited in search report | Publication date (day/month/year) | Patent family member(s) | Publication date (day/month/year) |
|---|---|---|---|
| WO 2022216920 A1 | 13 October 2022 | None | |
| CN 108251455 A | 06 July 2018 | None | |
| CN 104491877 A | 08 April 2015 | None | |
| WO 2013153092 A1 | 17 October 2013 | EP 2650013 A1 | 16 October 2013 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202311070972
- CN 202410074707
- WO 2022028462 A
- WO 2023274395 A
- WO 2023208023 A
- WO 2019089765 A1
- WO 2019161213 A